(19) Europäisches Patentamt

European Patent Office

Office européen des brevets

(11) **EP 0 511 306 B1**

(12) **EUROPEAN PATENT SPECIFICATION**

(45) Date of publication and mention
of the grant of the patent:
**17.07.2002  Bulletin 2002/29**

(21) Application number: **91903963.6**

(22) Date of filing: **17.01.1991**

(51) Int Cl.$^7$: **C07K 14/31**, A61K 38/16

(86) International application number:
**PCT/US91/00342**

(87) International publication number:
**WO 91/10680 (25.07.1991 Gazette 1991/17)**

(54) **TUMOR KILLING EFFECTS OF ENTEROTOXINS AND RELATED COMPOUNDS**

TUMOR-ZERSTÖRENDE EFFEKTE VON ENTEROTOXINEN UND VERWANDTEN VERBINDUNGEN

EFFETS DES ENTEROTOXINES ET DE COMPOSES APPARENTES POUR L'ELIMINATION DE TUMEURS

(84) Designated Contracting States:
**AT BE CH DE DK ES FR GB GR IT LI LU NL SE**

(30) Priority: **17.01.1990  US 466577**

(43) Date of publication of application:
**04.11.1992  Bulletin 1992/45**

(60) Divisional application:
**01102715.8 / 1 103 268**
**01104586.1 / 1 129 717**

(73) Proprietors:
• **TERMAN, David S.**
**Pebble Beach, CA 93953 (US)**
• **STONE, Jay L.**
**Aptos, CA 95003 (US)**

(72) Inventors:
• **TERMAN, David S.**
**Pebble Beach, CA 93953 (US)**
• **STONE, Jay L.**
**Aptos, CA 95003 (US)**

(74) Representative: **Wichmann, Hendrik, Dr. et al**
**Patent- und Rechtsanwälte**
**Bardehle - Pagenberg - Dost**
**Altenburg - Geissler - Isenbruck,**
**Galileiplatz 1**
**81679 München (DE)**

(56) References cited:
**WO-A-88/02632**

• **CELLULAR IMMUNOLOGY, vol. 97, no. 2, February 1986, pages 371-385; C.D. PLATSOUCAS et al.: "Immunomodulation of human leukocytes by staphylococcal enterotoxin A: augmentation of natural killer cells and induction of suppressor cells"**
• **Biological Abstracts, Volume 88, No. 7, issued 01 October 1989, SHCHEGLOVITOVA et al., "Effect of Staphylococcus enterotoxin a on the development of Lewis lung carcinoma in mice". See Abstract 75810, EKSP ONKOL, 11(1): 73-74.**
• **Biological Abstracts, Volume 88, No. 8, issued 10 October 1989, SHCHEGLOVITOVAL et al, "Effect of Staphylococcal enterotoxin A-sensitized spleen cells on the metastasizing of mouse Lewis lung carcinoma". See Abstract 87362, EKSP ONKOL, II(2): 54-57.**
• **Biological Abstracts, Volume 84, No. 5 issued 01 September 1987, SHCHEGLOVITOVA et al., "Nonspecific antitumor activity of staphylococcal enterotoxins". See Abstract 48345, EKSP ONKOL, 9(1): 28-30.**
• **Infection and Immunity, Vol. 57, No. 7, issued July 1989, PENARRUBIA et al., "Selective Proliferation of Nautral killer Cells among Monoate Depleted Peripheral Blood Mononuclear Cells as a Result of Stimulation with Staphylococcal Enterotoxin B" pgs. 2057-2065. See "introduction".**
• **Proc. Natl. Acad. Sci., Vol. 72, No. 9, issued September 1975, CARSWELL et al., "An endotoxin-induced serum factor that causes necrosis of tumors" pgs. 3666-3670. See entire document.**

- Infection and Immunity, Vol. 57, No. 1, issued January 1989, FAST et al. "Toxic Shock Syndrome Associated Staphylococcal and Streptococcal Pyrogenic Toxins are Potent Inducers of Tumor Necrosis Factor Production", pgs. 291-294. See Abstract.

- NATURE 339 (1989) 221-3; CELLULAR IMMUNOLOGY 122 (1989) 108-121 J EXP MED 171 (1990) 455-464; J IMMUNOL 144 (1990) 2473-9

**Description**

Technical Field

This invention relates generally to tumoricidal compositions and methods, and more specifically to the use of enterotoxins A, B, C, D, E and F (TSST-1) derived from, or homologous to those derived from Staphylococcus aureus, homologous synthetic polypeptides and derivatized enterotoxins.

Background Of The Invention

Therapy of the neoplastic diseases has largely involved the use of chemotherapeutic agents, radiation and surgery. However, results with these measures, while beneficial in some tumors, has had only marginal or no effect in many others, while demonstrating unacceptable toxicity. Hence, there has been a quest for newer modalities to treat neoplastic diseases.

In 1980, tumoricidal effects were demonstrated in four of five patients with advanced breast cancer utilizing therapy with plasma perfused over Staphylococcal Protein A. Terman, D.S., Young, J.B., Shearer, W.T., Ayus, C., Lehane, D., Mattioli, C., Espada, R., Howell, J.F., Yamamoto, T., Zaleski, H.E., Miller, L., Frommer, P., Feldman, L., Henry, J.F., Tillquist, R., Cook, G., Daskal, Y., New Eng. J. Med., 305, 1195, 1981. This elaborate system involved the administration of patient plasma which was perfused over a solid surface to which Staphylococcal Protein A was chemically attached. Protein A was prepared by batch fermentation of Staphylococcus. It was isolated from the media and partially purified by affinity chromatography.

While the initial observations of tumor killing effects with the immobilized Protein A perfusion system have been confirmed, additional results have been inconsistent. The explanation of these inconsistencies appears to be as follows. First, commercial Protein A has been shown to be an impure preparation, as evident from polyacrylamide gel electrophoresis studies. Secondly, various methods of the immobilization of Protein A to solid supports have been used, sometimes resulting in loss of biological activity of the plasma perfusion system. Thirdly, the plasma used for perfusion over the immobilized Protein A has been stored and treated in different ways, sometimes resulting in inactivation of the system. Moreover, the antitumor element present in this extremely complex perfusion system has not been previously defined. The system contained an enormous number of biologically active materials, to include Staphylococcal Protein A itself, Staphylococcal proteases, nucleases, exotoxins, enterotoxins and leukocidin, as well as the solid support and coating materials. Additional products included several anaphylatoxins generated in plasma after contact with immobilized Protein A. Finally, it is speculated that the biological activity of the system was due to extraction by Protein A of immunosuppressive immune complexes capable of blocking the host's antitumor response.

The present invention demonstrates that isolated Staphylococcal enterotoxins identified initially as contaminants in commercial Protein A can reliably reproduce the tumoricidal reactions and toxicity observed with the whole perfusion system. As such, these materials appear to represent the most active tumoricidal components in the Protein A perfusion system matrix. These materials demonstrate tumoricidal activity in small doses and produce tumoricidal effects and toxicity identical to that observed in animals and man with the whole Protein A perfusion system. However, the tumoricidal effects may be produced by a simple intravenous injection. Therefore, it has been possible to completely eliminate the elaborate and complex Protein A perfusion system, with its enormous number of component parts, unpredictable performance and potential toxicity problems. This system may be replaced by the enterotoxins which may be administered via simple intravenous injection and have the distinct advantages of convenience, reliability and safety over the cumbersome, inefficient and often ineffective extracorporeal Protein A perfusion system.

Enterotoxins have distinct advantages in inducing tumor killing effects over the more cumbersome and elaborate Staphylococcal Protein A plasma perfusion systems. One advantage is that enterotoxins are relatively simple proteins that may be infused after being solubilized in saline. This solubility obviates the need to immobilize Protein A or other biologicals on a solid support, and eliminates the requirement for perfusing plasma over a solid surface. Moreover, it bypasses problems associated with potential toxic reactions to impurities of Protein A. Consequently, enterotoxins appear to be far safer and more effective than previously described systems. Moreover, the system requires no elaborate sterilization and there is no problem with potential leaching of immobilized materials or chemical products from an inert surface as there would be with an extracorporeal column. Hence, this product offers decided advantages of effectiveness and convenience over the original system. Indeed, all evidence points to enterotoxins as being the most active antitumor product in the Staphylococcal Protein A plasma perfusion system.

WO 91/04053, cited as prior art under Art. 54(3) EPC only, is concerned with the treatment of cancers expressing MHC class II antigens only.

WO 92/01470, cited as prior art under Art. 54(3) EPC only, describes that a specific cell-killing effect mediated by cytotoxic T-cells (CTLs) can be achieved with superantigens, if they are covalently linked to an antibody directed against an epitope that is specific for the cell to be killed.

Hedlund *et al.*, Cellular Immunology 129, 426-434, 1990, reports that *Staphylococcal* enterotoxins direct human T lymphocytes to execute cytotoxicity toward MHC class II-expressing Raji cells, but not against MHC class II-deficient Raji mutant RJ 2.2.5.

Garcia-Penarrubia *et al.*, Infection and Immunity 57, 2057-2065, 1989, discloses that *Staphylococcus* enterotoxin B (SEB) stimulation of blood mononuclear cells resulted in selective proliferation of Natural Killer (NK) cells. However, it is also disclosed that SEB stimulation of blood cells (depleted of monocytes) resulted in an almost complete loss of T cells.

WO 98/09691 describes an extracorporeal system in which whole blood was exposed to SEs *ex vivo.* The SEs were then removed before the activated cells were administered.

## Summary Of The Invention

The present invention describes that enterotoxins derived from Staphylococcus aureus are useful by themselves for the treatment of cancer.

Accordingly, the use of an agent which is a *Staphylococcal aureus* enterotoxin (SE), or a homologue thereof or a fragment of an SE in the manufacture of a medicament for administration to a patient to treat carcinoma is provided as defined in the claims.

Enterotoxins are known to have molecular weights ranging from 22,000 to 38,000. They are heat stable, and resistant to trypsin digestion. According to one aspect of the present invention, enterotoxins isolated from media which is supporting the growth of various Staphylococcus aureus organisms are used in relatively pure form. When administered to subjects having tumors, the preparation induces a tumoricidal reaction: a hemorrhagic necrosis in tumor resulting in tumor regression. It should be understood that the term, "tumoricidal reaction," as used herein, means that the material under discussion promotes or assists in the killing of tumor cells.

Synthetic polypeptides with substantial structural homology and with statistically significant sequence homology and similarity to enterotoxin A, and enterotoxin B, including alignment of cysteine residues and similar hydropathy profiles, are also described as effective in tumoricidal therapy.

## Brief Description of Figures

Figure 1 shows the alignment of amino acid sequences of staphylococcal enterotoxins and their relatives.

Figure 2 shows the alignment of amino acid sequences of mature streptococcal pyrogenic exotoxin A and staphylococcus aureus enterotoxin B.

Figure 3 shows the hypothetical structure for the complex of Class II MHC, T cell receptor, and staphylococcal enterotoxins or Mls.

## Description Of The Specific Embodiments

The enterotoxins of Staphylococcus aureus form a group of serologically distinct extracellular proteins, designated A, B, $C_1$, $C_2$, $C_3$, D, E and F. These proteins are recognized as the causative agents of Staphylococcal food poisoning. Enterotoxin F appears to be important in the pathogenesis of the Staphylococcal toxic shock syndrome. Ingestion of preformed enterotoxin in contaminated food leads to the rapid development (within two to six hours) of symptoms of vomiting and diarrhea that are characteristic of Staphylococcal food poisoning.

The enterotoxin proteins are of similar molecular weight. Characteristically, they have a disulfide loop near the middle of the molecule, and are easily soluble in water and salt solutions. They are relatively resistant to proteolytic enzymes and to heat. The higher level structural similarities between the enterotoxins is in agreement with the clinical picture where all of the enterotoxins seem to produce similar effects of sepsis, hypotension and fever. General properties of the enterotoxins are given in Table 1.

TABLE 1A

| SOME PROPERTIES OF THE ENTEROTOXINS | | | | |
|---|---|---|---|---|
| | Enterotoxin | | | |
| | $A^a$ | $B^b$ | $C_1{}^c$ | $C_2{}^d$ |
| Emetic dose ($ED_{50}$)(μg/monkey) | 5 | 5 | 5 | 5-10 |
| Nitrogen content (%) | 16.5 | 16.1 | 16.2 | 16.0 |
| Sedimentation coefficient ($S_{20,w}$)(S) | 3.04 | 2.78 | 3.00 | 2.90 |
| Diffusion coefficient ($D_{20,w}$)(x $10^{-7}cm^2sec^{-1}$) | 7.94 | 8.22 | 8.10 | 8.10 |
| Reduced viscosity (ml/gm) | 4.07 | 3.81 | 3.4 | 3.7 |
| Molecular weight | 34,700 | 30,000 | 34,100 | 34,000 |
| Partial specific volume | 0.726 | 0.726 | 0.728 | 0.725 |
| Isoelectric point | 6.8 | 8.6 | 8.6 | 7.0 |
| Maximum absorption (mμ) | 277 | 277 | 277 | 277 |
| Extinction ($E_{1cm}^{1\%}$) | 14.3 | 14.4 | 12.1 | 12.1 |

[a] F.S., Thadhani, K., Schantz, E.J., Bergdoll, M.S., Biochemistry 5, 3281, 1966.

[b] Bergdoll, M.S., Borja, C.R., Avena, R.M., J. Bacteriol. 90, 1481, 1965.

[c] Borja, C.R., Bergdoll, M.S., Biochemistry 6, 1467, 1967.

[d] Avena, R.M., Bergdoll, M.S., Biochemistry 6, 1474, 1967.

TABLE 1B

| Physicochemical Properties of Staphylococcal Enterotoxins* | | | | | | |
|---|---|---|---|---|---|---|
| | Enterotoxin | | | | | |
| Property | $A^a$ | $B^b$ | $C_1{}^c$ | $C_2{}^d$ | $D^e$ | $E_f$ |
| Emetic dose for monkey (μg) | 5 | 5 | 5 | 5-10 | - | - |
| Sedimentation coefficient ($S_{20.w}$) | 3.03 | 2.89 | 3.0 | 2.9 | - | 2.6 |
| Molecular weight | 27,800 | 28,366[g] | 26,000 | 34,100 | 27,300 | 29,600 |
| Isoelectric point | 7.26 | 8.6 | 8.6 | 7.0 | 7.4 | 7.0 |
| C-terminal residue | Serine | Lysine | Glycine | Glycine | Lysine | Threonine |
| N-terminal residue | Alanine | Glutamic acid | Glutamic acid | Glutamic acid | Serine | - |

[a] Schantz, E.J., Roessler, W.G., Woodburn, M.J., Lynch, J.M., Jacoby, H.M., Silverman, S.J., Gorman, S.J., Biochemistry 11, 360, 1972.

[b] Schantz, E.J., Roessler, W.G., Wagman, J., Spero, L., Dunnery. D.A., Bergdoll, M.S., Biochemistry 4, 1011, 1965.

[c] Borje, C.R., Bergdoll, M.S., Biochemistry 6, 1467 (1967).

[d] Avene, R.M., Bergdoll, M.S. Biochemistry 6, 1474 (1967).

[e] Chang, P.C., Bergdoll, M.S., Biochemistry, 18, 1937, 1979.

[f] Borja, C.R., Fanning, E., Huang, I.Y., Bergdoll, M.S., J. Biol. Chem. 247, 2456, 1972.

[g] Dayhoff, M. ed. (1972) Data Section. in Atlas Protein Sequence Structure 5 : D227 National Biomedical Research Foundation, Washington, D.C, (determined from the amino acid sequence of Huang and Bergdoll, 1970). Huang, I.Y., Bergdoll, M.S., J. Biol. Chem. 245, 3493, 1970.

* Modified from Bergdoll, M.S., Czop, J.K., Gould, S.S., Enterotoxin Synthesis by the Staphylococci. In: Recent Advances In Staphylococcal Research, pp. 307-316, Yotis; W.W. (Ed.) Ann. N.Y. Acad. Sci. Vol. 236.

Amino acid compositions of enterotoxins A, B, $C_1$, $C_2$ and E reveal a high content of lysine, aspartic acid and tyrosine. Enterotoxins A and E are similar in methionine, leucine and arginine content, differing in this regard from enterotoxins B, $C_1$ and $C_2$. The amino acid sequence of enterotoxin B was found to consist of 239 amino acids. Half-cystine residues found at positions 92 and 112 form a disulfide bridge, and it has been suggested that the primary structure in this region may be common to all of the enterotoxins.

The protein sequences and immunological cross reactivity of the enterotoxins reveal that they can be divided into two related groups. SEA (Staphylococcal enterotoxin A), SEE and SED constitute one group, and SEB, SEC and Streptococcal pyrogenic exotoxin A (SPEA) make up the second group. Amino acid sequences show that SEA and SEE are almost identical and that SEB, SEC and SPEA share regions of similar sequence. SED is moderately related to both groups although it is more similar to the SEA group. There is a striking amino acid similarity among enterotoxins A, B, C, D and E in the region immediately downstream from cystine located at residue 106 in SEA. A second region at residue 147 also shows a highly conserved sequence. These regions are contained on the peptide fragment of SEC, shown to contain the active sites for emesis and diarrhea. The mitogenic region resides in the C terminal tryptic fragment of SEC, implying that other regions of sequence similarity exist. Amino acid sequence similarities and congruences are given in Tables 2-4.

## TABLE 2[*]

### SEQUENCE SIMILARITIES AMONG THE PYROGENIC TOXINS AND ENTEROTOXINS

| TOXIN | SEQUENCE | |
|---|---|---|
| | 106_____119 | 147_____163 |
| SEA | CMYGGVTLHDNNRL | KKNVTVQELDLQARRYL |
| SEB | CMYGGVTEHHGNOL | KKKVTAQELDYLTRHYL |
| SEC1 | CMYGGITKHEGNHF | KKSVTAQELDIKARNFL |
| SED | CTYGGVTPHEGNKL | KKNVTVQELDAQARRYL |
| SEE | CMYGGVTLHDNNRL | KKEVTVQELDLQARHYL |
| SPEA | CIYGGVTNHEGNHL | KKMVTAQELDYKVRKYL |
| Consensus | CMYGGVTLHEGNHL | KKNVTAQELD$\frac{L}{Y}$QAR$\frac{R}{H}$YL |
| TSST-1 | IHFQISGVTNTEKL | KKQLAISTLDFEIRHQL |

[*] Iandolo, J.J., Annu. Rev. Microbiol., 43, 375, 1989.

## TABLE 3

| Amino Acid Composition of the Enterotoxins (g 100g protein) | | | | | |
|---|---|---|---|---|---|
| | Enterotoxin | | | | |
| Amino Acid | A[*] | B[†] | C$_1$[‡] | C$_2$[‡] | E[§] |
| Lysine | 11.26 | 14.85 | 14.43 | 13.99 | 10.83 |

[*] Schantz et al., 1972.

[†] Bergdoll, M.S., Chu, F.S., Huang, I.Y., Rowe, C., Shih, T., Arch Biochem Biophys, 112, 104, 1965.

[‡] Huang , I.Y., Shih, T., Borja, C.R., Avena, R.M., Bergdoll, M.S., Biochemistry, 6, 1480, 1967.

[§] Borja et al., 1972.

[¶] From Bergdoll, M.S., Huang, I.Y., Schantz, E.J., J. Agric. Food Chem. 22, 9, 1974.

TABLE 3   (continued)

| Amino Acid Composition of the Enterotoxins (g 100g protein) | | | | | |
|---|---|---|---|---|---|
| | Enterotoxin | | | | |
| Amino Acid | A* | B† | C₁‡ | C₂‡ | E§ |
| Histidine | 3.16 | 2.34 | 2.91 | 2.87 | 3.04 |
| Arginine | 4.02 | 2.69 | 1.71 | 1.75 | 4.50 |
| Aspartic acid | 15.53 | 18.13 | 17.85 | 18.38 | 15.10 |
| Threonine | 5.96 | 4.50 | 5.31 | 5.80 | 6.36 |
| Serine | 2.99 | 4.05 | 4.58 | 4.81 | 4.72 |
| Glutamic acid | 12.36 | 9.45 | 8.95 | 8.93 | 12.15 |
| Proline | 1.35 | 2.11 | 2.16 | 2.23 | 1.93 |
| Glycine | 2.96 | 1.78 | 2.99 | 2.90 | 4.10 |
| Alanine | 1.94 | 1.32 | 1.85 | 1.61 | 2.38 |
| Half-cystine | 0.66 | 0.68 | 0.79 | 0.74 | 0.81 |
| Valine | 4.93 | 5.66 | 6.50 | 5.87 | 4.36 |
| Methionine | 0.96 | 3.52 | 3.20 | 3.60 | 0.45 |
| Isoleucine | 4.11 | 3.53 | 4.09 | 4.02 | 4.30 |
| Leucine | 9.78 | 6.86 | 6.54 | 6.13 | 10.08 |
| Tyrosine | 10.63 | 11.50 | 9.80 | 10.27 | 9.79 |
| Phenylalanine | 4.31 | 6.23 | 5.35 | 5.25 | 4.47 |
| Trytpophane | 1.46 | 0.95 | 0.99 | 0.84 | 1.51 |
| Amide $NH_3$ | 1.80 | 1.66 | 1.71 | 1.62 | 1.66 |
| TOTAL | 98.37 | 100.15 | 100.00 | 99.99 | 100.88 |

* Schantz et al., 1972.

† Bergdoll, M.S., Chu, F.S., Huang, I.Y., Rowe, C., Shih, T., Arch Biochem Biophys, 112, 104, 1965.

‡ Huang , I.Y., Shih, T., Borja, C.R., Avena, R.M., Bergdoll, M.S., Biochemistry, 6, 1480, 1967.

§ Borja et al., 1972.

¶ From Bergdoll, M.S., Huang, I.Y., Schantz, E.J., J. Agric. Food Chem. 22, 9, 1974.

TABLE 4†

| Amino Acid Compositions of TSST-1a and 1b[a] | | | |
|---|---|---|---|
| | Amino acid composition | | |
| Amino acid | TSST-1a residues per mole[b] | TSST-1b residues per mole[b] | TSST-1 clone[b] |
| Aspartic acid | 26 | 27 | 25 |
| Threonine | 21 | 20 | 19 |
| Serine | 20 | 20 | 21 |
| Glutamic acid | 20 | 20 | 17 |
| Proline | 10 | 8 | 10 |
| Glycine | 13 | 14 | 11 |
| Alanine | 4 | 5 | 3 |
| Half-cystine | 0 | 0 | 0 |
| Valine | 5 | 5 | 5 |
| Methionine | 0 | 0 | 2 |
| Isoleucine | 15 | 15 | 17 |
| Leucine | 14 | 16 | 15 |
| Tyrosine | 10 | 8 | 9 |
| Phenylalanine | 7 | 7 | 7 |

† Blomster-Hautamaa, D.A., Schlievert, P.M., Methods in Enzymology, 165, 37, 1988.

[a]Isolated from strain MN8, as compared to the inferred amino acid composition of the TSST-1 structural gene.

[b]Residues per mole values are based on a molecular weight of 22,000.

TABLE 4†   (continued)

| Amino Acid Compositions of TSST-1a and 1b[a] | | | |
|---|---|---|---|
| | Amino acid composition | | |
| Amino acid | TSST-1a residues per mole[b] | TSST-1b residues per mole[b] | TSST-1 clone[b] |
| Histidine | 5 | 5 | 5 |
| Lysine | 23 | 24 | 21 |
| Tryptophan | ND[d] | ND[d] | 3 |
| Arginine | 4 | 5 | 4 |
| | 197 | 199 | 194 |

† Blomster-Hautamaa, D.A., Schlievert, P.M., Methods in Enzymology, 165, 37, 1988.
[a]Isolated from strain MN8, as compared to the inferred amino acid composition of the TSST-1 structural gene.
[b]Residues per mole values are based on a molecular weight of 22,000.
[c]Residues per mole inferred from the DNA sequence of the TSST-1 structural gene. Blomster-Hautamaa and colleagues.
[d]ND. Not determined.

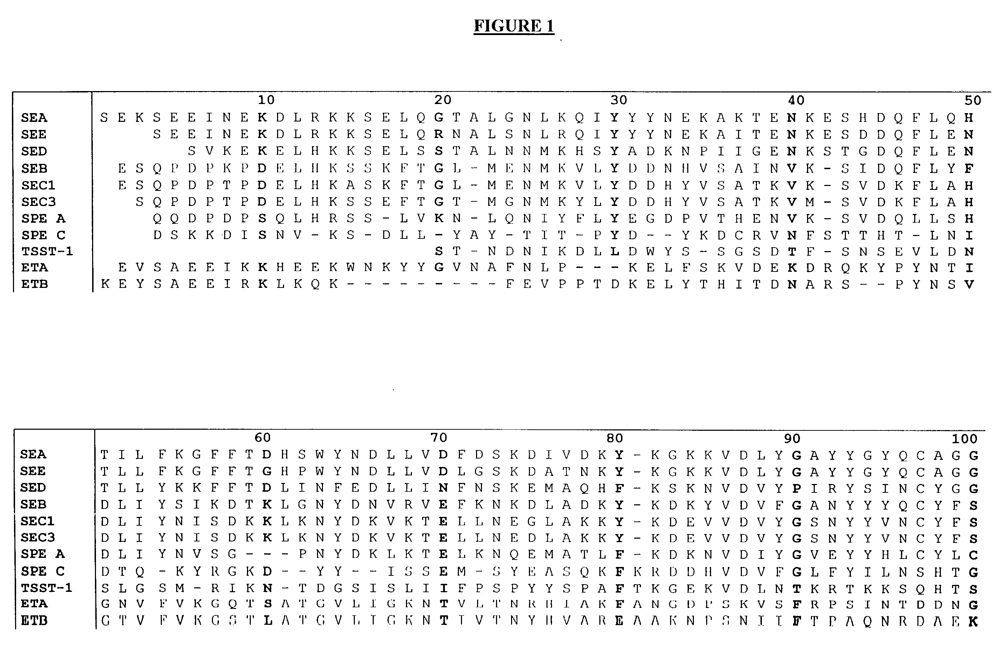

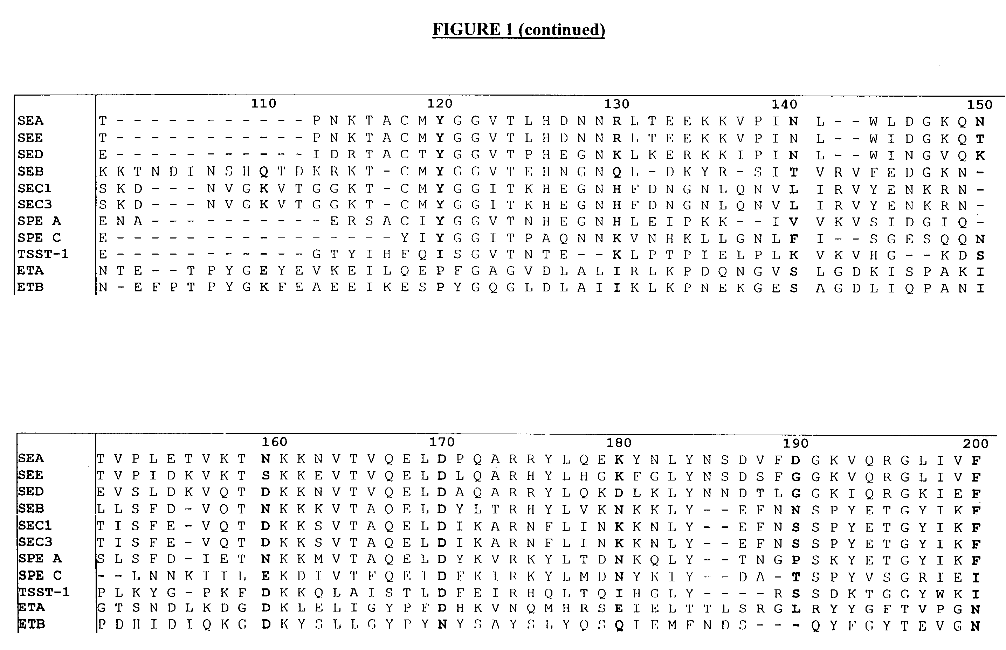

Comparison of the primary sequences of the staphylococcal enterotoxins and their relatives is shown in Figure 1. The complete primary amino acid sequences of the staphylococcal enterotoxins and related proteins are shown aligned, with the exception of the sequences of the exfoliating toxins, which are shown aligned, with the exception of the sequences of the exfoliating toxins, which are shown aligned with each other, but not with the remaining toxins. The exfoliating toxin sequences are shown here for completeness, and because these toxins have properties related to those of the others (see below). Toxins shown are as follows: SEA to SEE, Staphylococcus aureus enterotoxins A to E; SPE A and C, Streptococcus pyogenes toxins A and C; TSST1, Staphylococcus aureus toxic shock - associated toxin; ETA and ETB, Staphylococcus aureus exfoliating toxins A and B. Data are from (9-17). Residues that are identical or that have changed to an amino acid with similar properties among at least two of the following: SEA, SEE, and SED, are highlighted in pink. Residues that are identical or that have changed to an amino acid with similar properties among at least two of the following: SEB, SEC1, and SED and at least two of SEB, SEC1, and SEC3, are highlighted in yellow. Single letter abbreviations for the amino acid residues are: A, Ala; C, Cys; D, Asp; E, Glu; F, Phe; G, Gly; H, His; I Ile; K, Lys; L, Leu; M, Met; N, Asn; P, Pro; Q, Gln; R, Arg; S, Ser; T, Thr; V, Val; W, Trp; and Y, Tyr.
There is evidence that indicates varying degrees of immunological relatedness between certain enterotoxins. Bergdoll, M.S., Borja, C.R., Robbins, R., Weiss, K.F., Infect. Immun., 4, 593, 1971; Bergdoll, M.S., Enterotoxins. In: Staphylococci and Staphylococci Infections ed. C.S.F. Easmon, C. Adlam 1, pp. 559-598, 1983, Landon, Academic; Freer, J.H., Arbuthnott, J.P., Pharm. Ther., 19, 55, 1983. A considerable degree of cross reactivity exists for antisera raised against one enterotoxin and other enterotoxins. It has been considered that the enterotoxins may contain major cross reactive antigenic sites, while each individual enterotoxin possesses minor specific antigenic regions. Common precipitating antibodies were formed between SEA and SED. In addition, enterotoxins B and C can react immunologically with antisera against either toxin type. Immunologic cross reactivity between Streptococcal pyrogenic exotoxin A and Staphylococcal enterotoxins B and $C_1$ has been shown. These results suggest a conserved domain present in the three exotoxins. SEA, SEB, SEC, SED, TSST-1 and the pyrogenic exotoxins have also been shown to share considerable DNA and amino acid homology. The enterotoxins, the pyrogenic exotoxins and TSST-1 therefore appear to be evolutionarily related and all belong to a common generic group of proteins.
It should be noted that the two Streptococcal toxins SPEA and C are about as similar to each of the Staphylococcal groups as they are to each other. Exfoliative toxins are of similar size to SEB and SEA with similar modes of action. They share several points of sequence similarity to the Staphylococcal enterotoxins. Overall there are several stretches at which similarities are apparent throughout the total group comprised of Staphylococcal enterotoxins, Streptococcal pyrogenic exotoxins and Staphylococcal exfoliative toxins. The longest of these, located two-thirds of the way through the proteins, is similar to sequences found at the COOH-terminal end of the human and mouse invariant chain.
Invariant chain is a polypeptide associated with nascent MHC class II molecules. Class II molecules bind peptides and present them to T cells during immune responses. Indeed, many toxins bind to class II molecules. The shared sequences may indicate some or all of the invariant chain and toxin binding sites on class II molecules.
The known structural homology between the enterotoxins and Streptococcal pyrogenic exotoxin is further supported by the identity of clinical responses. It is known that this exotoxin induces hypotension, fever, chills and septic shock in man. It is hypothesized that this compound activates cytokines, such as interleukin 1, interleukin 2, tumor necrosis factor and interferon, and procoagulant activity which are the prime mediators of the clinical symptomatology. It is hypothesized that many other bacterial products are capable of inducing similar in vivo activity. Among potential tumoricidal agents which are likely candidates based upon structural homology or identity of clinical symptomatology are gram positive bacterial products, cell wall bacterial constituents such as peptidoglycans and various gram negative bacterial components to include meningococcal, pseudomonous and E. Coli products. While presently undemonstrated in animal systems, it is believed that these agents are likely to possess similar tumoricidal utility as those claimed here for the enterotoxins.

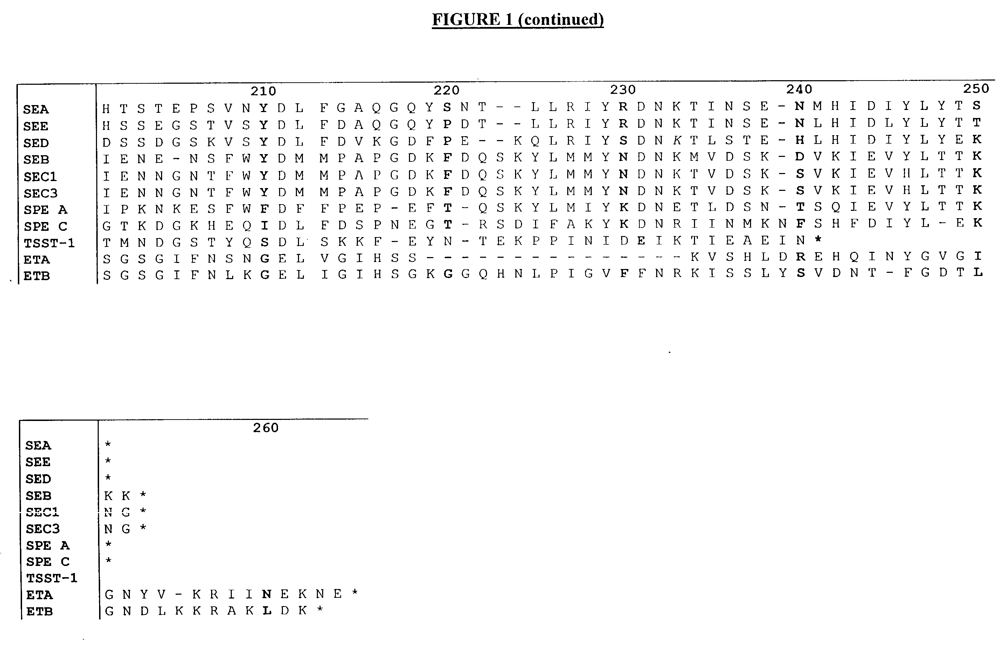

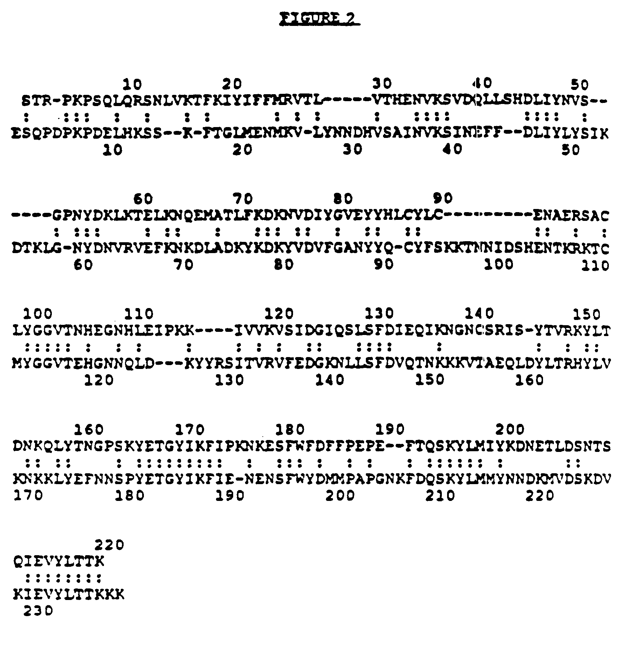

The recognition that the biologically active regions of the enterotoxins and SPEA were substantially structurally homologous enables one to predict synthetic polypeptide compounds which will exhibit similar tumoricidal effects. Figure 2 illustrates the amino acid sequence homology of mature SPEA and Staphylococcus aureus enterotoxin B. The top sequence is the SPEA-derived amino acid sequence. The amino acid sequence of enterotoxin B is on the bottom. Sequences are numbered from the amino acid terminus, with amino acids represented by standard one character designations. (See Tables 5 and 6 below.) Identities are indicated by : and gaps in the sequences introduced by the alignment algorithm are represented by dashed lines. See Johnson, L.P., L'Italien, J.J., and Schlievert, P.M., "Streptococcal pyrogenic exotoxin type A (scarlet fever toxins) is related to staphylococcus aureus enterotoxin B," Mol. Gen. Genet. (1986) 203: 354-356.

One common methodology for evaluating sequence homology, and more importantly statistically significant similarities, is to use a Monte Carlo analysis using an algorithm written by Lipman and Pearson to obtain a Z value. According to this analysis, a Z value greater than 6 indicates probable significance, and a Z value greater than 10 is considered to be statistically significant. Pearson, W.R., Lipman, D.J., "Improved tools for biological sequence comparison," Proc Natl Acad Sci U S A, April 1988, 85 (8) pages 2444-8; Lipman, D.J., Pearson, W.R., "Rapid and sensitive protein similarity searches," Science, March 22, 1985, 227 (4693) pages 1435-41.

In the present invention, synthetic polypeptides useful in tumoricidal therapy are characterized by substantial structural homology to enterotoxin A and enterotoxin B with statistically significant sequence homology and similarity (Z value of Lipman and Pearson algorithm in Monte Carlo analysis exceeding 10) to include alignment of cysteine residues and similar hydropathy profiles.

TABLE 5

| Amino Acid | One-letter Symbol |
|---|---|
| Alanine | A |
| Arginine | R |
| Asparagine | N |
| Aspartic acid | D |
| Cysteine | C |
| Glutamine | Q |
| Glutamic acid | E |
| Glycine | G |
| Histidine | H |
| Isoleucine | I |
| Leucine | L |
| Lysine | K |
| Methionine | M |
| Phenylalanine | F |
| Proline | P |
| Serine | S |
| Threonine | T |
| Tryptophan | W |
| Tyrosine | Y |
| Valine | V |

TABLE 6

```
              10        20              30        40        50
    STR-PKPSQLQRSNLVKTFKIYIFFMRVTL-----VTHENVKSVDQLLSHDLIYNVS--
    :   ::: :   :   : :        : : :     :   ::::      :::: :
    ESQPDPKPDELHKSS--K-FTGLMENMKV-LYNNDHVSAINVKSINEFF--DLIYLYSIK
              10        20              30        40        50

              60        70        80        90
    ----GPNYDKLKTELKNQEMATLFKDKNVDIYGVEYYHLCYLC---------ENAERSAC
        : ::: :   : ::   :   ::: ::   :   ::  ::        ::   :  :
    DTKLG-NYDNVRVEFKNKDLADKYKDKYVDVFGANYYQ-CYFSKKTNNIDSHENTKRKTC
        60        70        80        90        100       110

    100       110             120       130       140       150
    LYGGVTNHEGNHLEIPKK----IVVKVSIDGIQSLSFDIEQIKNGNCSRIS-YTVRKYLT
    :::::: :   : :      :      : : :   ::   :::: :           : :::
    MYGGVTEHGNNQLD---KYYRSITVRVFEDGKNLLSFDVQTNKKKVTAEQLDYLTRHMLV
    120       130             140       150       160

    160       170       180       190       200
    DNKQLYTNGPSKYETGYIKFIPKNKESFWFDFFPEPE--FTQSKYLMIYKDNETLDSNTS
    :: :: :    : :::::::::  :   :::  :  : :   : :::::::: :     ::
    KNKKLYEFNNSPYETGYIKFIE-NENSFWYDMMPAPGNKFDQSKYLMMYNNDKMVDSKDV
    170       180       190       200       210       220

          220
    QIEVYLTTK
    :::::::::
    KIEVYLTTKKK
    230
```

The enterotoxins are presumed to function by affecting emetic receptors in the abdominal viscera which stimulate the emetic and diarrheal response. These toxins also stimulate T lymphocyte mitogenicity, procoagulant, chemotactic activity, as well as cysteinyl leukotriene, lymphokine, serine protease and thromboglobulin production. Cytokines known to be induced by enterotoxins induce interferon, tumor necrosis factor, interleukins one and two. They suppress immune responses, augment natural killer cell cytotoxicity, enhance gram-negative endotoxic lethality and induce fever and hypotension. These additional properties are shared with the pyrogenic exotoxins of both Staphylococcus aureus and streptococcus pyrogenes and TSST-1. Synthetic polypeptides would also be expected to demonstrate similar responses.

The Staphylococcal enterotoxins A, B, C, D, E, toxic shock toxin (TSST-1), a product of mycoplasma arthritidis and Mls antigens provoke dramatic T cell responses. Staphylococcal enterotoxins are the most powerful T call mitogens known eliciting strong polyclonal proliferation at concentrations $10^3$ lower than such conventional T cell mitogens as phytohemagglutinin. SEA is the most potent T cell mitogen, stimulating DNA synthesis at concentrations of $10^{-13}$ to $10^{-16}$ M in the human system. All stimulate a large proportion of both murine and human CD4+ and CD8+ T cells. Activity of these mitogens is tightly restricted by the major histocompatibility complex (MHC) class II antigens. It is proposed that the Staphylococcal enterotoxins, streptococcal pyrogenic exotoxins, exfoliative toxins and a product of mycoplasma arthritidis bind directly to the T cell receptor and to class II MHC. These two structures are brought into contact, thus stimulating T cell activation via the $V_\beta$ region of the T cell receptor mimicking strong alloreactive response.

Many toxins have binding affinities for MHC class II molecules which are involved in stimulating T cells. For example, SEA has a Kd for human class II of about $3.2 \times 10^{-7}$ M, SEB of $10^{-6}$ M and TSST-1 of $10^{-7}$ M. SEA and SEB probably bind to the same site on class II because they cross compete for binding. Exfoliative toxins bind only weakly or not at all to class II. SEB and TSST-1 have different binding sites on class II molecules.

The structure of class II consists of two immunoglobulin-like domains located close to the cell membrane which supports a structure constructed from the $NH_2$ terminal regions of both polypeptides of the protein and comprise an extended β-pleated sheet supporting two alpha helices separated by a cleft. Peptides derived from foreign materials or from proteolysis of cell proteins normally lie in this groove. It is this complex of MHC and peptide that stimulates T cells bearing alpha and beta receptors. Bacterial toxins do not normally bind to MHC molecules by occupying this groove and therefore do not behave like conventional peptide-MHC binding antigens. Toxins bind to three different class II proteins, namely DR, DP, DQ (or murine I-A, I-E). SEB and TSST-1 bind to DR and DQ alleles but not to DP. Toxin-class II complexes stimulate T cells. Most toxins bind preferentially to DR class II proteins, less well to DQ and not at all to DP. Different DR alleles have different affinities for a few of the toxins most notably SEE. In the mouse, complexes of toxins plus I-E (murine DR equivalent) stimulate T cells more efficiently than complexes of toxins with I-A (murine DQ analog). There is also evidence for weak haplotype specificity, e.g., toxins bound to I-A$^k$ stimulate T cells less well than toxins bound to I-A$^d$ or I-A$^b$. Staphylococcus aureus toxins bind more efficiently to human class II proteins than to mouse. A likely location for toxin binding to MHC may be at the sides of class II where 2 wings, the ends of the β-pleated strands, extend to either side of the proteins.

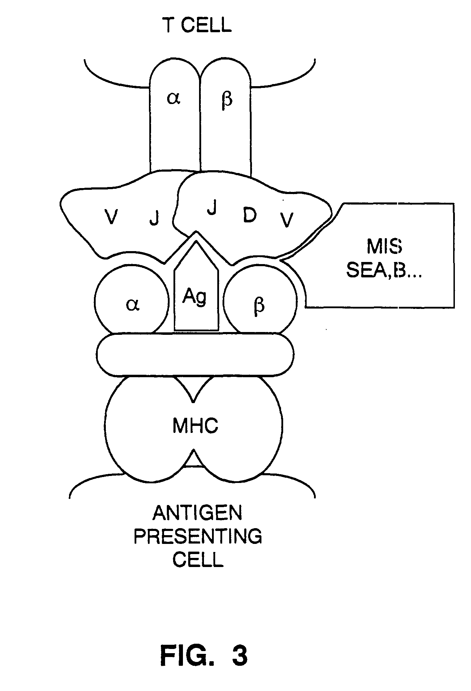

A hypothetical structure for the complex of class II MHC T cell receptor and Staphylococcal enterotoxins and MHC protein is given in Figure 3. The Figure shows a class II MHC protein, diagrammed according to Bjorkman and co-workers and Brown and co-workers, in contact with a T cell receptor and a staphylococcal enterotoxin or Mls product. Ag is the probable site of binding of a conventional antigenic peptide.

Toxins stimulate T cells through $V_\beta$ binding. T cell receptors for antigenic peptides bound to MHC proteins are made up of 5 clonally variable components $V_\alpha$, $J_\alpha$, $V_\beta$, $D_\beta$, and $J_\beta$. Recognition of most conventional antigenic peptides bound to MHC proteins involve contributions from all the variable components of the T cell receptor.

In contrast, the toxins stimulate T cells almost exclusively via the $V_\beta$ region of the T cell receptor. See Table 7 for binding of toxins to T cells bearing various $V_\beta$ receptors.

TABLE 7

| TOXIN | $V_\beta$ SPECIFICITY | |
|---|---|---|
| | HUMAN | MOUSE |
| SEA | ? | 1, 3, 10, 11, 17 |
| SEE | 5.1, 6.1-3, 8, 18 | 11, 15, 17 |
| SED | 5, 12, ? | 3, 7, 8.1-3, 11, 17 |
| SEB | 3, 12, 14, 15, 17, 20 | 3, 7, 8.1-3, 17 |
| SEC1 | 12, ? | 3, 8.2, 8.3, 11, 17 |
| SEC2 | 12, 13.1, 13.2, 14, 15, 17, 20 | 3, 8.2, 10, 17 |
| SEC3 | S, 12, ? | 3, 7, 8.1, 8.2 |
| TSST1 | 2 | 3, 15, 17 |
| ExFT | 2 | 3, 10, 11, 15, 17 |
| MAM | ? | 6, 8.1, 8.2, 8.3 |

This property of selective stimulation of $V_\beta$ is reminiscent of the endogenous superantigens called Mls antigens in the mouse. The pattern of $V_\beta$ Specificity of the different toxins corresponds loosely with their groupings by sequence similarity. SEA, SED and SEE all stimulate murine T cells bearing $V_\beta 11$ and SEE and SED both stimulate human T cells bearing $V_\beta 5$. SEB and SECs stimulate mouse T cells bearing members of the $V_\beta 8$ family and human T cells positive for $V_\beta 12$. The exceptions are as follows: SED stimulates T cells bearing the $V_\beta 8$ unlike SEA and SEE. Exfoliating toxin and TSST-1 which are not related by sequence have similar specificities for $V_\beta$ both in mouse and humans.

Bacterial toxins and other superantigens do not bind to T cell receptors at those regions involved in binding to conventional antigenic peptides plus MHC. The superantigens engage $V_\beta$ on an exposed face of $V_\beta$ or a region predicted to be a β-pleated sheet and exposed on the side of the T cell receptor. This model predicts that toxins act as clamps engaging the sides of class II and $V_\beta$ bringing into close proximity the surfaces of the T cell receptor and MHC that would contact each other during T cell recognition of conventional antigens bound in the groove of MHC. Proper confirmation must await x ray crystallographic resolution of the complex.

Neither class II nor toxins separately have affinities for the T cell receptors in question, but the combination of toxins and class II proteins do. only if the complex peptide-MHC ligand has formed can it functionally engage the T cell receptor. The T cell activation via the $V_\beta B$ region of the T cell mimics strong alloreactive responses. This interaction occurs irrespective of whether the $V_\beta$ is expressed on CD4+ or CD8+ T cells. This behavior is consistent with the known resistance of Staphylococcal enterotoxins to proteolysis even in acidified conditions.

Mice Express Endogenous Equivalent of the Enterotoxins.

T cells from some mice responded well to spleen cells from some other animals even though both responder and stimulator were identical at the MHC. The antigens are called minor lymphocyte stimulating antigens (Mls). There are many Mls-like products produced by mice controlled by non-linked loci. Mls products stimulate T cells bearing $V_\beta$S. Mls-1* in combination with mouse class II molecules stimulate nearly all T cells bearing mouse $V_\beta$ 6, 7, 8.1 and 9. A list of the Mls-like products and the $V_\beta$S they engage is given in Table 8. Mls products have not yet been found in humans.

TABLE 8

| Mls-like products identified in mouse. | | |
|---|---|---|
| LOCUS | $V_\beta$ specificity | MHC association |
| Mls-1[a] | 6, 7, 8.1, 9 | Class II (except q) |
| Mls-2[a] | 3 | Class II (except q) |
| Mls-3[a] | 3 | Class II (except q) |
| ? | 5 | I-E |
| ? | 7 | I-E |
| ? | 11 | I-E |
| ? | 17 | I-E |

A striking resemblance exists between T cell responses to Staphylococcal enterotoxins and T cell responses to the Mls locus. The Mls locus located on chromosome 1 and other similar genes on other (unknown) chromosomes have profound effects on T cells. Polymorphism at these loci elicits a strong primary mixed lymphocyte response between MHC identical and Mls disparate spleen cells in mice.

Mls products stimulate T cells bearing particular $V_\beta$5 almost regardless of the rest of the structure of the receptor on the T cell. This activity depends on the simultaneous expression by the presenting cell of class II proteins. Some class II products, most notably I-E molecules, present Mls products and bacterial toxins better than others. Mls appear to engage $V_\beta$S at the same site on the exposed face of the polypeptide as toxins.

The similarities between properties of bacterial toxins and mouse Mls products might lead one to suggest a structural similarity. Mls products associate with class II and stimulate T cells via $V_\beta$ much like superantigens but the structure of Mls is unknown.

There are consequences for mice expressing Mls products. They cause deletion in the thymus for all prospective T cells bearing $V_\beta$S with which they interact. Mice expressing Mls-1[a] contain very few T cells bearing $V_\beta$ 6, 7, 8.2 or 9 and hence are deprived of 20% of their total potential T cell repertoire. Despite this they do not seem to be susceptible to disease.

Both Mls and enterotoxins show the following characteristics in common:

1. Both activate a high frequency of normal T cells exceeding that of conventional protein antigens.
2. Responding T cells are CD4[+].
3. T cells of many specificities respond.
4. Both elicit responses of T cells expressing receptors having particular $V_\beta$ gene products.
5. There is no MHC restriction of responding T cells.
6. Both require presentation by class II MHC.
7. IE and IA molecules on antigen presenting cells are required for immunologic effects.
8. Ontogenetic deletion of $V_\beta$ or CD4[+]8[+] cells is induced by both molecules.

These similarities are summarized in Table 9.

TABLE 9

| Similarities between the T cell responses to Mls and SE and differences with responses to protein Ag[a] | | | |
|---|---|---|---|
| Characteristic of the T Cell Response to: | Mls-l[a] | SE | Proteins |
| High frequency of responding cells | Yes(~1:5) | Yes(~1:5) | No(~1:10[4]) |

[a]Data on T cell responses to Mls and SE derived from this paper and Janeway et al. A detailed description of the SE themselves is found in Bergdoll.

TABLE 9   (continued)

| Similarities between the T cell responses to Mls and SE and differences with responses to protein Ag[a] | | | |
|---|---|---|---|
| Characteristic of the T Cell Response to: | Mls-I[a] | SE | Proteins |
| Responding T cells CD4+ | Yes | Yes | Yes[b] |
| T cell receptor involved in response | Yes | Yes | Yes |
| T cells of many specificities respond | Yes | Yes | No |
| $V_\beta$ restriction of responding T cells | Yes | Yes | No[c] |
| MHC restriction of responding T cells | No | No | Yes |
| Incompetent class II MHC alleles | Yes | Yes | Yes[d] |
| 1-E more involved than 1-A | Yes | Yes | No |
| Ontogenetic deletion of $V_\beta$ on CD4+8+ | Yes | Yes[e] | No |
| Processing required | ? | No | Yes |
| Pulsing APC stimulatory | ? | Yes | Yes |
| Pulsing T cell stimulatory | ? | Yes | No |
| Protein identified | No | Yes | Yes |

[a]Data on T cell responses to Mls and SE derived from this paper and Janeway et al. A detailed description of the SE themselves is found in Bergdoll.

[b]T cells expressing CD8 respond only to proteins degraded within cells; extrinsic proteins are presented by class II MHC to CD4 T cells.

[c]T cell responses to protein antigens require all elements of the TCR, whereas those to Mls and SE appear to require only use of certain $V_\beta$ segments.

[d]Presentation of proteins is much more restricted in use of allelic forms of class II MHC molecules than is "presentation" of SE or Mls.

[e]From Yagi and Janeway.

The striking functional similarity of Staphylococcal enterotoxins and Mls suggests that the Mls may represent a protein with homology to Staphylococcal enterotoxins. It has been proposed that the Mls like Staphylococcal enterotoxins directly binds the TCR-CD4 complex via its $V_\beta$ domain and to class II MHC molecules assembling a complex that is highly stimulatory for T cells. Hence, both Mls and Staphylococcal enterotoxins are thought to ligate class II MHC to the TCR:CD4 complex in such a way as to stimulate a large percentage of T cells with restricted $V_\beta$ usage.

While the animal studies described herein were carried out with Staphylococcal enterotoxins A and B, based upon the observed structural and reactive similarities, it would be expected that similar results would be obtained with the other Staphylococcal enterotoxins, SPEA, TSST-1, mycoplasma arthritdisis, Mls antigens and the synthetic polypeptides described above. Additional biological properties common to this group include their mitogenic effects, interferon, interleukin and tumor necrosis factor induction activity. Furthermore, all are capable of inducing fever and shock when given intravenously to rabbits or monkeys, and most of these have been implicated as potential pathogenic agents in the toxic shock syndrome.

**Production And Isolation Of Enterotoxins A, B, C, D, E and F**

General Methods

Isolation and purification procedures for enterotoxins contain numerous common steps. On the whole, growth of enterotoxin producing Staphylococcus aureus strains is similar in all cases. The most widely used general medium for the culture of these organisms contains 3% MZ-amine Type A, or MAX, 3% protein hydrolysate powder, 0.00005% thiamine and 0.001% niacin. Optimum yields of the enterotoxins are obtained under controlled fermentation, where pH, temperature and oxygen tension are controlled. Typically, growth at 37°C for 18 to 24 hours is sufficient for maximum toxin yields. The yield of enterotoxin B and $C_1$ and $C_2$ will be up to several hundred μgrams (toxin)/ml (media), while the yield of other toxins will be only a few μgrams/ml.

All enterotoxins are secreted products. Generally, they are produced during the logarithmic and stationary stages of cell growth. After growth, the producing cells are removed from the medium by centrifugation, and the toxin-containing supernatant is saved. If a large fermentation has been carried out, then the cells and supernatant can be quickly separated using a continuous flow centrifuge. To concentrate the toxins from the media, various methods, e. g., polyethylene glycol precipitation, or dialysis tubing precipitation, or hollow fiber concentration using membranes with selective molecular weight cutoffs can be used. To assess the purity of the isolated enterotoxin product, specific antisera to each of the toxins are used in appropriate quantitative immunoassays, e.g., radioimmunoassays or enzyme labelled immunoassays, hemagglutination, or precipitin reactions.

**Enterotoxin Purification By Type**

Enterotoxin B

The strain of Staphylococcus aureus, that is used for the production of SEB (Staphylococcal enterotoxin B) is e.g., S6 or 10-275. (Source: Dr. John Iandolo, Kansas State University, Manhattan, Kansas.) The medium containing the toxin is diluted twice with water adjusted to a pH of 6.4, and AmberLite CG-50 (200 mesh) cation ion-exchange resin is added to the toxin mixture. The toxin is eluted, dialyzed, then reapplied to the CG-50 column again. The eluted toxin is dialyzed, then applied to a column of carboxymethyl cellulose or CM-Sephadex. Unbound proteins are eluted with 0.03 and 0.04 molar sodium phosphate buffer. At this point, the toxin is essentially homogeneous. Using chromatofocusing techniques, the SEB may be further subdivided into several isoelectric species using polybuffer 96.

Enterotoxin A (SEA)

High SEA producers, e.g., Staphylococcus aureus 13M-2909 (Source: Dr. John Iandolo, Kansas State University, Manhattan, Kansas) are grown in the general medium that is made 0.2% in glucose. Initially, AmberLite CG-50 is used for batch isolation. After incubation, the toxin is eluted and dialyzed. The toxin is then loaded onto a CM-cellulose column and eluted with a linear gradient. The combined fractions are then loaded onto a hydroxylapatite column and eluted using a linear gradient. The fractions are lyophilized and chromatographed on a Sephadex-G-75 column. The toxins obtained from this procedure are greater than 99% pure, with a yield of approximately 20%.

Enterotoxin $C_1$ ($SEC_1$)

Culture supernatant from Staphylococcus aureus 137 (Source: Dr. Marcia Betley, University of Wisconsin, Madison, Wisconsin) is concentrated, dialyzed and lyophilized. The toxin product is then applied to a carboxymethyl cellulose column and eluted with a stepwise gradient. The toxin peak consists of a sharp peak with a trailing edge. The eluted toxin is concentrated and applied to Sephadex-G-75. The toxin elutes as a single peak. The toxin is then concentrated and run twice through a column of Sephadex-G-50. The eluate is dialyzed against water and lyophilized.

Enterotoxin $C_2$ ($SEC_2$)

Culture supernatant from Staphylococcus aureus 361 (Source: Dr. Marcia Betley, University of Wisconsin, Madison, Wisconsin) is concentrated as for $SEC_1$ and dialyzed. The toxin is then applied to a carboxymethyl cellulose column. $SEC_2$ is eluted, lyophilized and resuspended in distilled water. The toxin is reapplied to a column of carboxymethyl cellulose and eluted with a linear gradient. The partially purified toxin is concentrated and applied to a Sephadex-G-75 column. The eluted toxin is concentrated and finally reapplied to a Sephadex-G-50 column. Recovery is about 40%, with purity exceeding 99%.

Enterotoxin D (SED)

Staphylococcus aureus 1151M (Source: Dr. John Iandolo, Kansas State University, Manhattan, Kansas) is used for the production of enterotoxin B. The medium is similar to that used for SEA and SEB. After growth and removal of the cells, the pH of the supernatant is adjusted to 5.6 and applied to an AmberLite-CG-50 resin. The mixture is stirred for one hour, and the toxin is eluted and concentrated using 20% (W/V) polyethylene glycol, 20M. The concentrated toxin is dialyzed and applied to a carboxymethyl cellulose column. The toxin is eluted in a linear gradient and then rechromatographed on carboxymethyl cellulose. The toxin solution is concentrated and chromatographed on Sephadex-G-75. This step is repeated once.

Enterotoxin E (SEE)

Staphylococcus aureus strain FRI-236 (Source: Dr. John Iandolo, Kansas State University, Manhattan, Kansas) culture supernatant is concentrated and dialyzed. The toxin is then absorbed to a carboxymethyl cellulose column. The toxin is eluted in a stepwise fashion and concentrated. It is then chromatographed twice on Sephadex-G-75. To obtain highly purified SEE, it is necessary to chromatograph the toxin once more on G-75 in the presence of 6 molar urea.

Enterotoxin F or Toxic Shock Syndrome Toxin-1 (TSST-1), TSST-1a and TSST-1b

Staphylococcus strain MN8 (Source: Dr. Patrick Schlievert, University of Minnesota, Minneapolis, Minnesota) is cultured overnight in dialyzable beef heart medium and precipitated from culture fluid by adding 4 volumes of absolute ethanol and storing for at least 2 days. The precipitate is collected by centrifugation and the pellet is suspended in water, recentrifuged and dialyzed to remove salts. The preparation is then electrofocused in a pH gradient of 3-10 using commercial ampholytes with the LKB Multiphor apparatus. The visible band containing TSST-1 is harvested and refocused in a pH 6-8 gradient yielding purified TSST-1.

TSST-1a and 1b are isolated by one additional electrofocusing step. After focusing TSST-1 on the pH 6-8 gradient, approximately one-half of the Sephadex gel is removed from the anode end. The gel remaining on the cathode end, containing the TSST-1 band is repoured after the addition of two more grams of Sephadex gel and then refocused overnight using the remaining pH gradient. After electrofocusing in a pH 6-8 or 6.5-7.5 gradient, protein bands are located by the zymogen print method. Discrete bands are scraped off the plate and eluted with pyrogen free water from the Sephadex gel. Strain MN8 yields approximately 2 mg of each toxin per liter of culture fluid. For Staphylococcus aureus strains other than MN8, 200 μg of each toxin is obtained per liter of culture fluid. TSST-1a and 1b are proteins which migrate as homogeneous bands in SDS gels to a molecular weight of 22,000 with isoelectric points of 7.08 and 7.22, respectively.

With the changing technology of protein purification, new methods have been employed for the purification of certain enterotoxins from Staphylococcus aureus. Some of these methods are given here.

Enterotoxins A and $C_2$

A 10 ml culture of Staphylococcus aureus 11N-165 (SEA), Staphylococcus aureus 361 (Source: Dr. John Iandolo, Kansas State University, Manhattan, Kansas) ($SEC_2$) is grown overnight at 37°C. The removal of enterotoxin from the supernatant is carried out using QAE-Sephadex. The toxin is then eluted batchwise from the ion exchanger and recovered by filtration on a sintered glass funnel. The eluates are concentrated by ultrafiltration. The toxin is then passed through a Sephadex-G-100 column. Two peaks absorbing at 280 mm are eluted, with the latter containing the enterotoxin. The eluted toxin is concentrated and rerun on Sephadex-G-100. The overall recovery is about 30% for $SEC_2$ and 40 to 50% for SEA. Both toxins appear homogeneous by sodium dodecylsulfate polyacrylamide gel electrophoresis.

Enterotoxins A, $C_1$, D

This method utilizes fast protein liquid chromatography (FPLC) and high resolution chromatofocusing Mono P column. Enterotoxins in media are concentrated and passed over a Sephadex-G-75 column. The toxin containing fractions are pooled. For $C_1$ and D, the supernatants are passed over an AmberLite-CG-50 column, as described for SED, and the active fractions pooled. All three toxins are then placed in buffer for chromatofocusing and then separated using the MONO P column FPLC system. Since all of the toxins have isoelectric points in the range of 7 to 9, the polybuffer PBE-96 is used for elution. The purity of SEA, $SEC_1$ and SED is estimated to be 98, 95 and 80%, respectively. SEA elutes as two peaks at pH 8.8 and 8.6. $SEC_1$ also elutes as two peaks at pH 8.3 and 7.9, and SED elutes as three peaks at pH 8.6, 8.3 and 8.0.

Enterotoxins may also be produced in mutant strains of Staphylococcus aureus by expression of an enterotoxin producing gene in another bacteria or cell. Genetic material which appears to be in the chromosomal plasmid, or phage portion of the bacteria may be used for gene insertion procedures. Complete molecules or fragments with amino acid sequence homology to the parent enterotoxin may be produced with this technology. (Reviewed in Iandolo, J.J., Annu. Rev. Microbiol., 43, 375, 1989.) Moreover, mutagenic agents such as N-Nitroso compounds are capable of augmenting significantly the production of enterotoxins by some strains of Staphylococcus.

Alpha Toxin

Staphylococcus aureus Wood 46 strain (Source: Dr. Sidney Harshman, Vanderbilt University, Nashville, Tennessee) is used and cultured in yeast extract dialysate medium. With the glass-pore bead method undialyzed yeast may be used together with casein, glucose, thiamin and nicotinic acid. The organism is incubated in medium for 24h at 37°C.

The culture supernatant is applied to a glass-pore bead column and adjusted to pH 6.8. A column of 5 x 20 cm is used for 3 liter batches and flow rates adjusted to 10-20 ml/min. The column is washed with 0.01M $KHPO_4$ pH 6.8 and then the alpha toxin is eluted with 1.0M $KHPO_4$ pH 7.5. Fractions are tested for the presence of alpha hemolysin by a rapid hemolytic assay using rabbit erythrocytes as substrate.

Streptococcal Pyrogenic Exotoxin (SPE)

(Erythrogenic toxin, scarlet fever toxin)

Streptococcus NY-5 strain (Source: ATCC 12351) has been the most widely used for toxin production and studies. A list of various strains to produce toxins A, B, and C has been published. The Kalbach S84 type 3 strain (Source: Dr. Joseph E. Alouf, Institute Pasteur-Unite Associee, Paris, France) is cultured and the supernatant is concentrated and stirred in calcium phosphate gel. Fraction $S_1$ is precipitated with 80% saturated ammonium sulfate. The redissolved pellet is dialyzed and termed Fraction $S_2$. This fraction is precipitated with 50-80% ammonium sulfate, resuspended in phosphate buffered saline (Fraction $S_3$), and gel filtered on a Bio-Gel P-100 column. The fraction corresponding to the volume eluted between 160 and 240 ml is collected and concentrated by ultrafiltration to about 20 ml in an Amicon PM10 Membrane (Fraction $S_4$). Fraction $S_4$ is then submitted to preparative isoelectric focusing (IEF) performed with a 100 ml column. The material which focuses at around pH 4.8 in a narrow peak is collected and dialyzed in an Amicon cell using PBS to eliminate ampholines and sucrose. The Fraction ($S_5$) constitutes purified pyrogenic exotoxin. Another electrophoretic form of SPE with a pI of 4.2 is often separated simultaneously with that of pI 4.8. Both forms show total cross reactivity against immune sera raised by rabbit immunization with fraction $S_3$.

The Fraction $S_5$ shows a single band by SDS-PAGE corresponding to a molecular weight of 28K. Bioassays for determination of activity include erythematosus skin test in rabbits or guinea pigs lymphocyte blast transformation. The toxin may also be detected by enzyme-linked immunoabsorbent assay (ELISA) or hemagglutination inhibition.

**Experimental Animal studies**

1. Enterotoxins

Staphylococcus aureus strain FRI-722 was the production strain for SEA and strain 110-275 was the production strain for SEB. Both were grown in 3% enzyme hydrolyzed casein and 1% yeast extract at pH 6.6 at a temperature of 35-37°C for 16-20 hours. The culture medium is then filtered and incubated with AmberLite CG50 and the bound protein eluted with high ionic strength buffer (impure preparations). For further purification, this step is repeated and elution carried out with a low ionic strength to high ionic strength buffer gradient. The fraction containing SEB is concentrated, dialyzed and loaded onto a molecular sieve gel filtration column. The fraction containing SEB is concentrated and dialyzed against phosphate buffered saline pH 7.2, filter sterilized and frozen. Samples containing 5 and 10 mg/ml are tested in double diffusion immunoprecipitation assay using known standards of SEB and SEA with monospecific antisera. A single precipitation line was noted which showed a line of identity with known SEB and SEA, respectively. For enterotoxin B batches, polyacrylamide gel electrophoresis showed bands at 28,000 and a weaker band at 20,000 molecular weight. Purified enterotoxin A showed only a single band at 28,000 molecular weight.

High performance liquid chromatography (HPLC) profiles were obtained on a MAC PLUS controlling a Rainin Rabbit HPLC with a Hewlett Packard 1040 A Diode array detector and a Vyadac Protein and Peptide C18 column. The profile for purified enterotoxin B was a sharp peak without significant shoulder. There was minimal trace contamination. A functional hemolytic assay for the presence of alpha hemolysin in the pure preparation was negative. Purified enterotoxin batches were negative for endotoxin in the limulus amebocyte lysate assay. The sterility of the preparations was demonstrated by negative cultures in thioglycolate medium and soybean-casein digest. protein determinations were carried out by a spectrophotometric method.

The functional activity of the toxins in vitro was tested using an established system of mitogenicity. Human peripheral blood lymphocytes $5\times10^5$/well, were incubated in flat bottomed microtiter plates and stimulated with graded doses of exotoxins for various periods. Each well was pulsed with 1uc [$^3$H] thymidine 18 hours before termination of the cultures which were harvested and processed for liquid scintillation counting.

2. Animals

New Zealand white female rabbits weighing from 2.5 to 5.0 kg, ages 2 to 4 months were used for studies employing purified enterotoxins. Rabbits of higher weight were used in the preliminary studies which are discussed in application Serial No. 07/416,530, filed on October 3, 1989. The animals were obtained from the Elkhorn Rabbitry, Watsonville, California.

3. Tumor

The tumor used for these studies was obtained from the Frederick Cancer Research Facility of the National Cancer Institute. It was stored frozen in the DCT tumor repository. The tumor call lettered G50014 was also known as the VX-2. Stewart, H.L., Snell, K.C., Dunham, L.J. : Transplantable and transmissible tumors of animals. In Atlas of Tumor Pathology. Washington, D.C, Armed Forces Institute of Pathol., pp. 38, 355, 1959. The tumor is a carcinoma indigenous to the New Zealand white rabbit. It was stored as a tissue fragment, and suspended in saline. The tumor was initially induced by Shope virus and derived from a transformed papilloma in a dutch belted rabbit. Kidd and Rous described the tumor in 1937. Histopathologically, the tumor consists of cords and sheets of epithelial cells (80%) and 20% hemorrhage and necrosis with no acini. The growth is primarily papillary. Numerous mitoses are evident. The cells are thin walled and very anaplastic. The tumor used was cryopreserved from October 20, 1985. It had a negative viral profile.

4. Tumor inoculation

Tumor fragments for inoculation were obtained from VX-2 growing in rabbit thigh. Fragments were implanted intramuscularly into the right thigh of recipients. Donors were placed under general anesthesia with halothane (1.5%) and under sterile conditions, small fragments were excised and placed in Dulbecco's Modified Eagles Medium with glutamine (Gibco Life Technologies, Inc., Grand Island, NY 14072). The fragments were rinsed and then suspended in media until they were transferred into new hosts. Recipient rabbits had their right thigh shaved and scrubbed with alcohol and betadine. A small area was anesthetized with 1% lidocaine. With a scalpel, an incision was made through the skin into the muscle where a small pocket was created. With forceps, 4 to 5 tumor fragments were implanted into the muscle. The wound was closed with 1 or 2 nylon sutures. Tumors appeared at the implantation site within 4 weeks and therapy was started when the tumors were at least 1 to 2 centimeters in broad diameter.

5. Tumor measurements.

Tumors were measured by calipers by a certified veterinary oncologist before and at intervals after treatment. Complete remission was present when there was no evident tumor. Partial remission represented a reduction of tumor volume by greater than 50%. Less than partial remission was a 25-50% reduction in tumor volume.

6. Conditions of Administration

Enterotoxins A and B in lyophilized form were diluted in 0.9% saline and then filtered through a 0.45 micron Millipore filter. Aliquots were stored at -20° F. An aliquot was thawed once, used only for a single injection and then discarded. Enterotoxin preparations in appropriate dose were prepared in 1 ml of 0.9% saline and drawn up in a 1 ml syringe. This solution was administered via the central ear vein which was cannulated with a 25 gauge needle and attached infusion tubing (Butterfly, 25 x 3/4 with 12" tubing set, Abbott Hospital, N. Chicago, IL 50064). The tubing and needle were initially washed with saline using a 3 ml syringe and with the tubing filled with saline, the toxin infusion was begun using a 1 ml tuberculin syringe (Monoject tuberculin 1.0 cc, Division of Sherwood Medical, St. Louis 63103). Approximately 0.3 ml of toxin was administered per minute. The tubing and needle were washed with 6 ml of normal saline over an additional 3 minutes using a 3 ml syringe. Partially purified enterotoxin B in doses of 100-150 μg enterotoxin/kg body weight or 40-60 μg enterotoxin/kg body weight were injected. Purified enterotoxin B was used in doses of 13-40 μg enterotoxin/kg body weight and purified enterotoxin A was used in doses of 0.5-12 μg enterotoxin/Kg body weight.

7. Enterotoxin Administration to Tumor Bearing Rabbits.

Preliminary studies in 20 rabbits using partially purified enterotoxin B as a single dose of 100-150 μg/kg or 40-60 μg/kg resulted in tumor regressions. With a dose of 40-60 μg/kg, six of twelve animals showed objective tumor regressions while a dose of 100-150 μg/kg resulted in objective tumor responses in three of nine rabbits treated. Results of these studies are given in prior application, Serial No. 07/466,577, filed on January 17, 1990. Toxicity of these preparations was thought to be due to contaminating elements in particular staphylococcal alpha hemolysin. Accordingly, the next phase of these studies was carried out with purified enterotoxin B.

a) Purified Enterotoxin B

Purified enterotoxin B in a mean dose of 26 μg/kg was administered to seven animals on one, two or three occasions (Table 10). Five showed complete remissions while one additional rabbit demonstrated 96% regression. One showed tumor progression. Of the four animals receiving a mean dose of 13 μg/kg, one had a complete remission while three showed tumor progression. A single animal given a dose of 40 μg/kg died within 12 hours of injection. Six of eight animals with major regressions showed enduring responses lasting 2 to 6 months without evident tumor recurrence (Table 10).

b) Purified Enterotoxin A

SEA in a dose of 0.5 µg/kg was given to 5 rabbits on two or three occasions. One showed a complete remission and a second 93.5% remission while two others demonstrated tumor progression and one died acutely after the third injection. SEA in a dose range of 5-12 µg/kg was administered to 7 animals. Two achieved complete remission while one experienced a 60% remission. Four others died acutely after the first injection.

c) Purified TSST-1

One rabbit was treated with 0.5 µg/kg of TSST-1 and showed a complete remission over 40 days.

d) Untreated Control Animals

Five animals were inoculated with the VX-2 carcinoma as given above but were not treated with enterotoxins. All five showed progressive tumor growth over 90 days observation. No spontaneous remissions of tumor were observed.

TABLE 10

| Purified Enterotoxin B (Lot TTB-16) | | |
|---|---|---|
| Animal Number | Maximum Response | Time to Maximum Response (days) |
| Mean Dosage 26 µg/kg | | |
| QT[2] | complete remission | 24 |
| Wanda[1] | complete remission | 20 |
| Cindy[2] | complete remission | 30 |
| Edna[2] | complete remission | 46 |
| Magnolia[3] | complete remission | 75 |
| Periwinkle[3] | 96% | 68 |
| Heidi[2] | progression | |
| Mean Dosage 13 µg/kg | | |
| KT[1] | complete remission | 14 |
| Dinky[2] | progression | |
| Mazie[2] | progression | |
| Gretta[1] | progression | |
| Mean Dosage 40 µg/kg | | |
| Bonnie[1] | NC | 12 hours (acute death) |

[1] One injection on day 0.

[2] Two injections: One injection on day 0 and one injection on days 4, 5, 7 or 8.

[3] Three injections: One injection on day 0, one injection on days 4 or 6, and one injection on days 11 or 13.

8. Histology

Microscopically, tumors showed extensive hemorrhagic necrosis in samples obtained 12 to 72 hours after the initial injection. Control untreated tumor showed focal areas of necrosis within the tumor, but no areas of hemorrhagic necrosis. Indeed, the areas of necrosis were far more extensive in the treated tumors with few if any areas of viable tumor. In the treated tumors, small blood vessels demonstrated hemostasis, and focal areas of inflammatory cell extravasation in the perivascular area. These changes were not seen in control untreated tumor specimens.

9. Toxicity

Acute toxic effects were observed in all animals after enterotoxin administration, to include temperature elevations of 2 to 5°F and anorexia. The animals became subdued. At the same time, the tumors became soft and mobile. There were signs of diarrhea in several animals. Toxicity of partially purified enterotoxin B is given in copending patent application Serial No. 07/416,530, filed October 3, 1989.

a) <u>Purified Enterotoxins A and B</u>

With purified enterotoxin B, the dose range of 5-26 µg/kg was associated with fever and anorexia, while the 40 µg/kg dose was lethal in one animal tested. Five of seven rabbits given enterotoxin A in doses of 5-12 µg/kg died within 72 hours of the first dose. However, when the dose was reduced to 0.5 µg/kg four of five animals survived with two showing tumor regressions and one dying after the third injection. The surviving animals showed temperature elevations of 2° to 5° F and anorexia for 1-3 days after injection. During acute inflammatory activity in the tumor, animals often lost weight.

10. Multiple injections of enterotoxins induce antitumor effects

Tumor bearing rabbits were given two or three injections of SEB, SEA or TSST-1 and showed tumor regressions. It is known that enterotoxins induce production of various cytokines and that one such cytokine namely interferon will in turn upregulate the surface expression of IA molecules and Class II major histocompatibility antigens. Such additional upregulated antigen presenting cells, would be further capable of binding additional enterotoxins and presenting them to the T lymphocyte repertoire. Moreover, a synergy has been noted between various cytokines namely tumor necrosis factor, interferon and various mitogens for T lymphocyte activation. Therefore, we may speculate that in the presence of various cytokines induced by the first injection of enterotoxins, upregulated antigen presenting cells are primed to bind additional toxin given in the second or third injection producing substantially augmented T cell proliferative responses and associated anti-tumor effects.

It is conceivable that the enterotoxins night be employed together with various cytokines such as IL-2 <u>in vitro</u> to develop a highly enriched population of T lymphocytes that could subsequently be injected at various intervals to continuously augment the anti-tumor effect in tumor bearing hosts.

Finally, while the enterotoxins were given intravenously in the present experiments, it is quite conceivable that the toxins could be administered in adjuvant form bound to vehicles such as aluminum hydroxide, liposomes, water in oil emulsions, pluronic triblock polymers and saponin with similar anti-tumor effects.

11. Attenuation of toxicity with Ibuprofen

The administration of Ibuprofen (20 mg/ml) given in doses of 0.25 to 0.5 ml subcutaneously when temperatures reached 105°F or greater resulted in reduction in fever by 2 to 5°F. Ibuprofen could be administered every 4 to 6 hours; however, in general, it did not need to be given more than once or twice per 24 hours. The use of this drug did not interfere with the observed tumor reduction or histologic hemorrhagic necrosis.

Ibuprofen may inhibit the prostaglandin mediated effects of the inflammatory cytokines including fever and anorexia but does not affect other antitumor immune and inflammatory responses.

Ibuprofen is only one of a large group of drugs known as non-steroidal anti-inflammatory agents (cyclooxygenase and prostaglandin synthesis inhibitors), which would also be useful to attenuate toxicity induced by the enterotoxins.

12. Genetic Aspects of Enterotoxin Production

Proceeding from the seminal work of Cohen & Boyer, U.S. Patent No. 4,237,224, DNA technology has become useful to provide novel DNA sequences and produce large amounts of heterologous proteins in transformed cell cultures. In general, the joining of DNA from different organisms relies on the excision of DNA sequences using restriction endonucleases. These enzymes are used to cut donor DNA at very specific locations, resulting in gene fragments which contain the DNA sequences of interest. These DNA fragments usually contain short single-stranded tails at each end, termed "sticky-ends". These sticky-ended fragments can then be ligated to complementary fragments in expression vehicles which have been prepared, e.g., by digestion with the same restriction endonucleases. Having created an expression vector which contains the structural gene of interest in proper orientation with the control elements, one can use this vector to transform host cells and express the desired gene product with the cellular machinery available. Once expressed, the gene product is generally recovered by lysing the cell culture, if the product is expressed intracellularly, or recovering the product from the medium if it is secreted by the host cell.

Recombinant DNA technology has been used to express entirely heterologous gene products, termed direct expression, or the gene product of interest can be expressed as a fusion protein containing some parts of the amino acid sequence of a homologous protein. This fusion protein is generally processed post-translationally to recover the native gene product. Many of the techniques useful in this technology can be found in Maniatis, T., et al., Molecular Cloning: A Laboratory Manual, Cold Spring Harbor Laboratory, New York (1982).

However, while the general methods are easy to summarize, the construction of an expression vector containing a desired structural gene is a difficult process and the successful expression of the desired gene product in significant amounts while retaining its biological activity is not readily predictable. Frequently gene products are not biologically active when expressed in yeast, bacteria or mammalian cell systems. In these cases, post-translational processing is required to produce biological activity.

From physical and genetic analysis, the genes for SEA, SEB, SEC, and SEE occupy a chromosomal loci. The structural gene encoding SED in all strains examined is localized, to a large penicillinase-like plasmid.

The enterotoxin A gene has been cloned. SEA was expressed in the E. coli genetic background from a single 2.5 kbp Hind III chromosomal DNA fragment. When sequenced, the DNA was found to contain a single reading frame that generated a protein consistent with the partial sequences of SEA derived by chemical methods. Therefore, it is apparent that the site mapped contained the structural gene for SEA. Betley, M.J., Mekalanos, J.J., J. Bacteriol, 170, 34, 1987; Huang, I.Y., Hughes, J.L., Bergdoll, M.S., Schantz, E.J., J. Biol. Chem., 262, 7006, 1987; Betley, M., Lofdahl, S., Kreiswirth, B.N., Bergdoll, M.S., Novick, R.P. Proc. Natl. Acd., Sci., USA, 81, 5179, 1984.

The enterotoxin A gene was found to be at least 18 kilobases in length and was carried on a mobile element. Enterotoxin A production was linked to the presence of a bacteriophage which integrates into the bacterial chromosome. The enterotoxin A gene is located near the phage attachment. The enterotoxin A gene was mapped between the purine and isoleucine-valine markers in 24 Staphylococcus aureus strains. Conversion to the SEA producing phenotype was induced by lysogenization with a temperate phage purified from staphylococcal aureus strain PS42D. Therefore, a bacteriophage vector was found to be responsible for the toxin phenotype in suitable recipients.

The enterotoxin B gene has been cloned and expressed in E. Coli. The DNA of the gene derived from E. Coli has been sequenced and matches the chemically derived sequence with only minor differences. Gaskill, M.E., Khan, S.A., J. Biol. Chem., 263, 6276, 1988; Jones, C.L., Khan, S.A., J. Bacteriol, 166, 29, 1986; Huang, I.Y., Bergdoll, M. S., J. Biol. Chem., 245, 3518, 1970.

The SEC gene has been cloned from the chromosome of Staphylococcus aureus MN Don. The cloned toxin was expressed in E. coli with a molecular weight comparable to that of the toxin from Staphylococcus aureus. The toxin was biologically active as measured by pyrogenicity, enhancement of lethal endotoxic shock and mitogenicity with murine splenocytes. The DNA sequence of the enterotoxin C gene has been developed and a protein sequence derived that compares favorably with the complete chemical sequence reported earlier. Bohach, G.A., Schlievert, P. M., Infect Immun., 55, 428, 1987; Bohach, G.A., Schlievert, P.M. Mol. Gen. Genet. 209, 15, 1987.

The enterotoxin D gene has been found to occur on a 27.6 kbp plasmid. The enterotoxin D gene has been cloned and expressed in E. coli and other Staphylococcal strains. The enterotoxin D gene in Staphylococcus aureus is under control of the agar locus like most Staphylococcal extracellular protein genes. The DNA sequence was determined encoding a mature protein with amino acid composition and reaction with antibody to SED confirming its identity to the biochemically purified toxin. Couch, J.L., Saltis, M.T., Betley, M.J., J. Bacteriol. 170, 2954, 1988.

The enterotoxin E gene has been cloned from S. Aureus FR1918 and was expressed in E. coli encoding an extracellular protein of 26,425 daltons. Its identity to SEE was confirmed immunologically and by correspondence of N terminal and C terminal analysis. Kreiswirth, B.N., Lofdahl, S., Betley, M.J., O'Reilly, Schlievert, P.M. Nature, 305, 709, 1983.

TSST-1 gene was not associated with either bacteriophage or plasmid DNA. The gene was cloned on a 10.6 kbp fragment of chromosomal DNA and subsequently on an approximately 1 kbp subclone of the larger fragment. The TSST-1 gene was expressed in E. Coli, and TSST-1 was secreted into the periplasm. The genetic element coding for TSST-1 was found to occupy two loci on the Staphylococcus genome. The loci are indicated by the notation Hi 555; one is at the junction of regions 1 and 2 and is indistinguishable from att012 and closely linked to tyrB. The second is within the trp operon at the junction of regions 17 and 18. Hi555 encodes the tst gene and is a heterologous insertion element that provisionally exhibits some of the characteristics of a transposon. Strains that are Trp⁻ contain Hi555 at regions 17 and 18 (linked to Trp), while strains that are Trp⁺ contain Hi555 elsewhere linked to TryB. However, the Trp⁻ phenotype is not due to insertional inactivation by the unusual element. The sequence and analysis of the tst gene has been described. It codes for a mature protein (TSST-1) of 197 amino acids and a molecular weight of 22,049. Cooney, J., Mulvey, M., Arbuthnott, J.P., Foster, T.J., J. Gen. Microbiol., 134, 2179, 1988.

Streptococcal pyrogenic exotoxin (SPEA) is clearly related to the enterotoxins. It has a cysteine loop of 9 amino acids similar to that of SEA and is also encoded by a converting phage. SPEA shows greater amino acid sequence similarity with SEB than SEA. Immunologic studies show that the proteins and antisera to either enterotoxin are cross reactive. Therefore, genes for all of the enterotoxins have been isolated and transfected into other bacteria to obtain selective production. These genes may be used as sources of accelerated production of these toxins in high producing bacteria employing transfection techniques familiar to one skilled in the art. Iandolo, J.J., Annu. Rev. Microbiol., 43, 375, 1989.

High producing strains of Staphylococcus for selective enterotoxin production have been identified and are available as described in enterotoxin purification section above. Moreover, exposure to mutagenic agents such as N-methyl-N'-nitro-N-nitrosoguanidine of enterotoxin producing Staphylococcus aureus has resulted in a 20 fold increase in enterotoxin production over the amounts produced by the parent Staphylococcus aureus strain. Freedman, M.A., Howard, M.B., J. Bacteriol, 106, 289, 1971.

13. Transfections of Tumor Cells With Enterotoxin Genes

The genes for the enterotoxins and streptococcal pyrogenic exotoxins have been cloned. With their known mimicry of the Mls locus and their affinity for T cell beta receptors, it would be logical to assume that transfection of the enterotoxin gene into tumor cells bearing appropriate HLA-DQ or DR or DP would result in production of a tumor cell bearing the minor lymphocyte stimulating locus capable of ligating MHC class II molecules with T lymphocytes, therefore stimulating potent T cell proliferation and associated antitumor immunity.

14. Bacterial Products Related to Staphylococcal Enterotoxins With Similar Biological Effects

Streptococcal pyrogenic exotoxin (SPE) is produced by many strains of group A streptococci. Three antigenically distinct types (A, B, C) have been described. It is now known that Streptococcal pyrogenic exotoxin or scarlet fever toxin is related to Staphylococcus aureus enterotoxin B. The amino acid sequence of SPE has significant homology with Staphylococcus aureus enterotoxin B but not with other proteins in the Dayhoff library. Figure 2 shows the alignment of amino acid sequences of mature SPEA and Staphylococcus aureus enterotoxin B, as reported in Johnson, L.P., L'Italien, J.J. and Schievert, P.M. "Streptococcal pyrogenic exotoxin type A (Scarlet fever toxin) is related to Staphylococcus aureus enterotoxin B," Mol. Gen. Genet (1986) 203:354-356.

The biological properties of SPE are shared with some Staphylococcal enterotoxins such as lymphocyte mitogenicity, fever induction and enhanced susceptibility to endotoxin shock when given intravenously. SPE activates murine T cells mainly $V_\beta 8.2$ in physical association with MHC class II molecules expressed on accessory cells. SPE causes deregulation of the immune response in vitro resulting in delayed (12-16 days) acceleration of humoral and cellular immune activity. This may account for the sustained anti-tumor responses noted with the use of its structural analog, namely enterotoxin B, when administered to rabbits with the VX2 carcinoma as demonstrated herein. Moreover, SPE has now been shown to induce a toxic shock like syndrome identical to that associated with various enterotoxins. Given the biological and structural relatedness of these proteins, it would be anticipated that SPE and any other protein, bacterial or otherwise, with homology to enterotoxins would produce tumoricidal effects identical to those of enterotoxins.

15. Staphylococcal Enterotoxin Protein Fragments With Biologic Activity

Studies of amino acid homology of Streptococcal pyrogenic exotoxin and enterotoxin B have suggested that there may be biologically active fragments present within the whole molecule. Indeed, cyanogen bromide generated toxin fragments of TSST-1 have been shown to be responsible for T lymphocyte mitogenicity and suppression of immunoglobulin synthesis. These functions could be selectively blocked by monoclonal antibodies directed to the respective fragments. Amino acid analysis of the toxins show that they contain similar domains that may give rise to mitogenic and emetic properties in susceptible cells. A peptide fragment in SEC was shown by Spero and Morlock to contain the active sites for emesis and diarrhea. The mitogenic region resided in the C terminal tryptic fragment of SEC.

An immune functional site on Staphylococcal enterotoxin A has been identified corresponding to residues 1-27 of SEA which is responsible for stimulation of T cell proliferation and induction of interferon-y. This SEA (1-27) sequence corresponds to N-Ser-GIv-Lys-Ser-Glu-Glu-Ile-Asn-GFlu-Lys-Asp-Lev-Arg Lys-Lys-Ser-Glu-Leu-Gln-Gly-Thr-Ala-Lev-Gly-Asn-Lev-Ly and blocks SEA induced T cell proliferation and production of interferon y which was not seen with SEA (28-48) peptide. Thus, a functional site on SEA responsible for modulation of T cell function involves the N-terminal 27 amino acids. These molecules may interact at either the level of TCR or the binding of SEA to class II MHC antigens.

For TSST-1, mitogenic activity was shown to be located on a 14,000 dalton cyanogen bromide generated toxin fragment. Other studies using proteolytic digestion of the TSST-1 with papain demonstrated mitogenic activity in 12,000 dalton fragment occupying 2/3 of TSST-1 molecule toward COOH terminal end of holotoxin. On the other hand, non-specific mitogenicity of rabbit lymphocytes demonstrated by enterotoxins A, B, and $C_1$ was associated with the $NH_2$ terminal ends of the molecules.

The emetic reaction and a related immediate-type skin reaction to SEB appears to be mediated by histamine and cysteinyl leukotrienes liberated from mast cells. Enterotoxins probably act on intramucosal or intradermal ganglion cells and the effect on mast cells is indirectly mediated by neuropeptides. Carboxymethylation of histidine residues of SEB caused a complete loss of emetic and skin sensitizing activity without changing the immunological specificity, e. g., T cell stimulating activity. An anti-idiotype monoclonal antibody against the combining site of an anti-SEB monoclonal antibody had no enterotoxic activity but can inhibit the enterotoxic activity, e.g., emetic response and diarrhea of a 10,000 molar excess of SEB. Anti-idiotype antibody also inhibited immediate-type skin reactions as well. The anti-idiotype antibody and carboxymethylated enterotoxins may be useful tools to protect against the enterotoxin induced intestinal toxicity.

Therefore, it could be predicted that fragments of the whole enterotoxin molecule can produce biologically active effects and predictably reproduce the in vivo tumoricidal activity of the whole molecule while eliminating some of the toxic effects noted.

Moreover, it would be reasonable to assume that similar or increased tumoricidal effects could be accomplished with biologically active fragments or intact enterotoxins alone or attached to antigen presenting cells (class II MHC, HLA-DR) and incubated ex vivo with a random T cell population or one which may have been pre-enriched for the appropriate $V_\beta$ receptor. The activated T cell population with bound enterotoxin might then be reinfused into the host. Similar tumoricidal effects would be anticipated with enterotoxins or biologically active fragments infused into a host who has had an "organoid" (an enriched T lymphocyte organ) implanted on a biocompatible matrix and placed in a site in the host such as the abdominal cavity, adjacent to the liver or subcutaneously.

Although the foregoing invention has been described in detail for purposes of clarity of understanding, certain modifications may be practiced within the scope of the appended claims. 'While the above findings apply to an experimental animal model, it should be recognized that the tumor used herein is an excellent model of human cancer. Therapeutic success in the canine model with PACC system (described in a series of patent applications, the latest of which is identified as Serial No. 331,095), the forerunner of the present invention, was transferred to humans in which objective tumor regressions were obtained in four of the first five consecutive patients treated. Thus, the data given herein for rabbits with carcinoma is expected to be predictive of success when the compositions are applied to humans with spontaneous tumors as well.

## Claims

1. Use of an agent which is a *Staphylococcal aureus* enterotoxin (SE), or a homologue of an SE that stimulates T cells via the TCR Vβ region and having a Z value of Lipman and Pearson algorithm in Monte Carlo analysis exceeding 10, or a fragment of an SE, the homologue or the fragment having essentially the same biological activity as an SE, a conjugate of SE with a monoclonal antibody specific for a cell surface structure on a carcinoma target cell lacking detectable expression of MHC class II or expressing marginal amounts of MHC class II proteins against which carcinoma cell unconjugated SE does not activate cytotoxic T cells being excluded, in the manufacture of a medicament for administration to a patient to treat carcinoma, the treatment of MHC class II expressing carcinomas being excluded.

2. Use according to claim 1, wherein the agent is selected from: SEA, SEB, SEC1, SEC2, SEC3, SED, SEE and SEF.

3. Use according to claim 1 or claim 2, wherein the medicament is designed to provide between 0.5 µg agent/kg body weight and 150 µg agent/kg body weight.

4. Use according to any one of claims 1 to 3, wherein the agent is said fragment of an SE.

5. Use according to claim 4, wherein the agent is said fragment of an SE selected from: SEA, SEB, SEC1, SEC2, SEC3, SED, SEE and SEF.

6. Use according to any one of claims 1 to 5, wherein the agent has a sequence as set out below:

CMYGGVTLHDNNRL

CMYGGVTEHHGNOL

CMYGGITKHEGNHF

CTYGGVTPHEGNKL

CMYGGVTLHDNNRL

CMYGGVTLHEGNHL

IHFQISGVTNTEKL.

7. Use according to any one of claims 1 to 5, wherein the agent has a sequence as set out below:

KKNVTVQELDLQARRYL

KKKVTAQELDYLTRHYL

KKSVTAQELDIKARNFL

KKNVTVQELDAQARRYL

KKEVTVQELDLQARHYL

KKNVTAQELD (L or Y) QAR (R or H)YL

KKQLAISTLDFEIRHQL.

8. Use according to any one of the preceding claims, wherein the medicament is presented as a preparation for single injection.

9. Use according to any one of claims 1 to 8, wherein the medicament is presented as a preparation for multiple injections.

10. Use according to any one of the preceding claims, wherein the medicament also comprises one or more of:

aluminium hyroxide

liposomes

a water-in-oil emulsion

pluronic triblock polymers

saponin

11. Use according to any one of the preceding claims, wherein the medicament also comprises a toxicity-attenuating dosage of a substance which is a non-steroidal, anti-inflammatory agent.

12. Use according to claim 11, wherein the non-steroidal, anti-inflammatory agent is ibuprofen.

**Patentansprüche**

1. Verwendung eines Agens, das ein Enterotoxin von *Staphylococcus aureus* (SE) ist, oder eines Homologs eines SEs, das T-Zellen über die TCR-Vβ-Region stimuliert und einen Z-Wert nach dem Algorithmus von Lipman und Pearson in einer Monte Carlo-Analyse besitzt, der 10 überschreitet, oder eines Fragment eines SEs, wobei das Homolog oder das Fragment im Wesentlichen die gleiche biologische Aktivität wie ein SE besitzen; wobei weiterhin ein Konjugat von SE mit einem monoklonalen Antikörper ausgeschlossen ist, der für eine Zelloberflächenstruktur auf einer Karzinom-Zielzelle spezifisch ist, der eine nachweisbare Expression von MHC-Klasse II-Proteinen fehlt oder die geringfügige Mengen an MHC-Klasse II-Proteine exprimiert und wobei gegen die Karzinomzelle unkonjugiertes SE nicht cytotoxische T-Zellen aktiviert; zur Herstellung eines Arzneimittels zur Verabreichung an einen Patienten zur Behandlung eines Karzinoms; wobei die Behandlung von MHC-Klasse IIexprimierenden Karzinomen ausgeschlossen ist.

2. Verwendung nach Anspruch 2, wobei das Agens ausgewählt ist aus: SEA, SEB, SEC1, SEC2, SEC3, SED, SEE und SEF.

3. Verwendung nach Anspruch 1 oder Anspruch 2, wobei das Arzneimittel so gestaltet ist, dass es zwischen 0,5 μg Agens/kg Körpergewicht und 150 μg Agens/kg Körpergewicht bereitstellt.

4. Verwendung nach einem der Ansprüche 1 bis 3, wobei das Agens das Fragment eine SEs ist.

5. Verwendung nach Anspruch 4, wobei das Agens das Fragment eines SEs ausgewählt aus SEA, SEB, SEC1, SEC2, SEC3, SED, SEE und SEF ist.

6. Verwendung nach einem der Ansprüche 1 bis 5, wobei das Agens eine Sequenz wie nachfolgend ausgeführt besitzt.

CMYGGVTLHDNNRL

CMYGGVTEHHGNOL

CMYGGITKHEGNHF

CTYGGVTPHEGNKL

CMYGGVTLHDNNRL

CMYGGVTLHEGNHL

IHFQISGVTNTEKL.

7. Verwendung nach einem der Ansprüche 1 bis 5, wobei das Agens eine Sequenz wie nachfolgend ausgeführt besitzt:

KKNVTVQELDLQARRYL

KKKVTAQELDYLTRHYL

KKSVTAQELDIKARNFL

KKNVTVQELDAQARRYL

KKEVTVQELDLQARHYL

KKNVTAQELD (L oder Y) QAR (R oder H)YL

KKQLAISTLDFEIRHQL.

**8.** Verwendung nach einem der vorangehenden Ansprüche, wobei das Arzneimittel als eine Zubereitung für eine Einzelinjektion präsentiert wird.

**9.** Verwendung nach einem der Ansprüche 1 bis 8, wobei das Arzneimittel als eine Zubereitung für Mehrfachinjektionen präsentiert wird.

**10.** Verwendung nach einem der vorangehenden Ansprüche, wobei das Arzneimittel auch einen oder mehrere der folgenden Bestandteile umfasst:

Aluminiumhydroxid
Liposomen
eine Wasser-in-Öl-Emulsion
Pluronic-Triblockpolymere
Saponin.

**11.** Verwendung nach einem der vorangehenden Ansprüche, wobei das Arzneimittel auch eine toxizitätsabschwächende Dosierung einer Substanz umfasst, die ein nicht-steroidales, anti-inflammatorisches Agens ist.

**12.** Verwendung nach Anspruch 11, wobei das nicht-steroidale, antiinflammatorische Agens Ibuprofen ist.

**Revendications**

**1.** Utilisation d'un agent qui est une entérotoxine de *Staphylococcal aureus* (SE), ou un homologue d'une SE qui stimule des cellules T via la région Vβ de TCR et ayant une valeur Z de l'algorithme de Lipman et Pearson lors d'une analyse de Monte Carlo dépassant 10, ou un fragment d'une SE, l'homologue ou le fragment ayant essentiellement la même activité biologique qu'une SE, un conjugué de SE avec un anticorps monoclonal spécifique à une structure de surface cellulaire sur une cellule cible de carcinome présentant une manque d'expression détectable de protéines MHC classe II ou exprimant des quantités marginales de protéine MHC classe II et contre ladite cellule de carcinome une SE non-conjuguée n'active pas des cellules T cytotoxique étant exclus, dans la fabrication d'un médicament pour l'administration à un patient afin de traiter un carcinome, le traitement de MHC classe II exprimant des carcinomes étant exclus.

**2.** Utilisation selon la revendication 1, dans laquelle l'agent est sélectionné parmi : SEA, SEB, SEC1, SEC2, SEC3, SED, SEE et SEF.

**3.** Utilisation selon la revendication 1 ou 2, dans laquelle le médicament est conçu pour délivrer entre 0,5 µg d'agent/kg de poids corporel et 150 µg d'agent/kg de poids corporel.

**4.** Utilisation selon l'une quelconque des revendications 1 à 3, dans laquelle l'agent est ledit fragment d'une SE.

**5.** Utilisation selon la revendication 4, dans laquelle l'agent est ledit fragment d'une SE sélectionné parmi : SEA , SEB, SEC1, SEC2, SEC3, SED, SEE et SEF.

**6.** Utilisation selon l'une quelconque des revendications 1 à 5, dans laquelle l'agent a une séquence telle qu'établie ci-dessous :

```
CMYGGVTLHDNNRL
CMYGGVTEHHGNOL
CMYGGITKHEGNHF
CTYGGVTPHEGNKL
CMYGGVTLHDNNRL
CMYGGVTLHEGNHL
IHFQISGVTNTEKL.
```

**7.** Utilisation selon l'une quelconque des revendications 1 à 5, dans laquelle l'agent a une séquence telle qu'établie ci-dessous :

```
KKNVTVQELDLQARRYL
KKKVTAQELDYLTRHYL
KKSVTAQELDIKARNFL
KKNVTVQELDAQARRYL
KKEVTVQELDLQARHYL
KKNVTAQELD(L ou Y)QAR(R ou H)YL
KKQLAISTLDFEIRHQL.
```

**8.** Utilisation selon l'une quelconque des revendications précédentes, dans laquelle le médicament est présenté sous forme d'une préparation pour une injection unique.

**9.** Utilisation selon l'une quelconque des revendications 1 à 8, dans laquelle le médicament est présenté sous forme d'une préparation pour des injections multiples.

**10.** Utilisation selon l'une quelconque des revendications précédentes, dans laquelle le médicament comporte également un ou plusieurs éléments suivants :

de l'hydroxyde d'aluminium
des liposomes
une émulsion eau dans huile
des polymères triblocs pluroniques
une saponine.

**11.** Utilisation selon l'une quelconque des revendications précédentes, dans laquelle le médicament comporte également un dosage d'atténuation de toxicité d'une substance qui est un agent anti-inflammatoire non-stéroïdien.

**12.** Utilisation selon la revendication 11, dans lequel l'agent anti-inflammatoire non-stéroïdien est l'ibuprofène.

# FIGURE 1

EP 0 511 306 B1

```
                        10             20             30             40             50
SEA     S E K S E E I N E K D L R K K S E L Q G T A L G N L K Q I Y Y Y N E K A K T E N K E S H D Q F L Q H
SEE         S E E I N E K D L R K K S E L Q R N A L S N L R Q I Y Y Y N E K A I T E N K E S D D Q F L E N
SED             S V K E K E L H K K S E L S S T A L N N M K H S Y A D K N P I I G E N K S T G D Q F L E N
SEB     E S Q P D P K P D E L H K S S K F T G L - M E N M K V L Y D D N H V S A I N V K - S I D Q F L Y F
SEC1    E S Q P D P T P D E L H K A S K F T G L - M E N M K V L Y D D H Y V S A T K V K - S V D K F L A H
SEC3      S Q P D P T P D E L H K S S E F T G T - M G N M K Y L Y D D H Y V S A T K V M - S V D K F L A H
SPE A     Q Q D P D P S Q L H R S S - L V K N - L Q N I Y F L Y E G D P V T H E N V K - S V D Q L L S H
SPE C     D S K K D I S N V - K S - D L L - Y A Y - T I T - P Y D - - Y K D C R V N F S T T H T - L N I
TSST-1                    S T - N D N I K D L L D W Y S - S G S D T F - S N S E V L D N
ETA     E V S A E E I K K H E E K W N K Y Y G V N A F N L P - - - K E L F S K V D E K D R Q K Y P Y N T I
ETB     K E Y S A E E I R K L K Q K - - - - - - - - - F E V P P T D K E L Y T H I T D N A R S - - P Y N S V
```

```
                        60             70             80             90            100
SEA     T I L F K G F F T D H S W Y N D L L V D F D S K D I V D K Y - K G K K V D L Y G A Y Y G Y Q C A G G
SEE     T L L F K G F F T G H P W Y N D L L V D L G S K D A T N K Y - K G K K V D L Y G A Y Y G Y Q C A G G
SED     T L L Y K K F F T D L I N F E D L L I N F N S K E M A Q H F - K S K N V D V Y P I R Y S I N C Y G G
SEB     D L I Y S I K D T K L G N Y D N V R V E F K N K D L A D K Y - K D K Y V D V F G A N Y Y Y Q C Y F S
SEC1    D L I Y N I S D K K L K N Y D K V K T E L L N E G L A K K Y - K D E V V D V Y G S N Y Y V N C Y F S
SEC3    D L I Y N I S D K K L K N Y D K V K T E L L N E D L A K K Y - K D E V V D V Y G S N Y Y V N C Y F S
SPE A   D L I Y N V S G - - - P N Y D K L K T E L K N Q E M A T L F - K D K N V D I Y G V E Y Y H L C Y L C
SPE C   D T Q - K Y R G K D - - Y Y - - I S S E M - S Y E A S Q K F K R D D H V D V F G L F Y I L N S H T G
TSST-1  S L G S M - R I K N - T D G S I S L I I F P S P Y Y S P A F T K G E K V D L N T K R T K K S Q H T S
ETA     G N V F V K G Q T S A T G V L I G K N T V L T N R H I A K F A N G D P S K V S F R P S I N T D D N G
ETB     G T V F V K G S T L A T G V L I G K N T I V T N Y H V A R E A A K N P S N I I F T P A Q N R D A E K
```

**FIGURE 1 (continued)**

```
                          110           120           130           140           150
SEA    T - - - - - - - - - - P N K T A C M Y G G V T L H D N N R L T E E K K V P I N L - - W L D G K Q N
SEE    T - - - - - - - - - - P N K T A C M Y G G V T L H D N N R L T E E K K V P I N L - - W I D G K Q T
SED    E - - - - - - - - - - I D R T A C T Y G G V T P H E G N K L K E R K K I P I N L - - W I N G V Q K
SEB    K K T N D I N S H Q T D K R K T - C M Y G G V T E H N G N Q L - D K Y R - S I T V R V F E D G K N -
SEC1   S K D - - - N V G K V T G G K T - C M Y G G I T K H E G N H F D N G N L Q N V L I R V Y E N K R N -
SEC3   S K D - - - N V G K V T G G K T - C M Y G G I T K H E G N H F D N G N L Q N V L I R V Y E N K R N -
SPE A  E N A - - - - - - - - - E R S A C I Y G G V T N H E G N H L E I P K K - - I V V K V S I D G I Q -
SPE C  E - - - - - - - - - - - - - - Y I Y G G I T P A Q N N K V N H K L L G N L F I - - S G E S Q Q N
TSST-1 E - - - - - - - - - - G T Y I H F Q I S G V T N T E - - K L P T P I E L P L K V K V H G - - K D S
ETA    N T E - - T P Y G E Y E V K E I L Q E P F G A G V D L A L I R L K P D Q N G V S L G D K I S P A K I
ETB    N - E F P T P Y G K F E A E E I K E S P Y G Q G L D L A I I K L K P N E K G E S A G D L I Q P A N I


                          160           170           180           190           200
SEA    T V P L E T V K T N K K N V T V Q E L D P Q A R R Y L Q E K Y N L Y N S D V F D G K V Q R G L I V F
SEE    T V P I D K V K T S K K E V T V Q E L D L Q A R H Y L H G K F G L Y N S D S F G G K V Q R G L I V F
SED    E V S L D K V Q T D K K N V T V Q E L D A Q A R R Y L Q K D L K L Y N N D T L G G K I Q R G K I E F
SEB    L L S F D - V Q T N K K K V T A Q E L D Y L T R H Y L V K N K K L Y - - E F N N S P Y E T G Y I K F
SEC1   T I S F E - V Q T D K K S V T A Q E L D I K A R N F L I N K K N L Y - - E F N S S P Y E T G Y I K F
SEC3   T I S F E - V Q T D K K S V T A Q E L D I K A R N F L I N K K N L Y - - E F N S S P Y E T G Y I K F
SPE A  S L S F D - I E T N K K M V T A Q E L D Y K V R K Y L T D N Q L Y - - T N G P S K Y E T G Y I K F
SPE C  - - L N N K I I L E K D I V T F Q E I D F K I R K Y L M D N Y K I Y - - D A - T S P Y V S G R I E I
TSST-1 P L K Y G - P K F D K K Q L A I S T L D F E I R H Q L T Q I H G L Y - - - - R S S D K T G G Y W K I
ETA    G T S N D L K D G D K L E L I G Y P F D H K V N Q M H R S E I E L T T L S R G L R Y Y G F T V P G N
ETB    P D H I D I Q K G D K Y S L L G Y P Y N Y S A Y S L Y Q S Q T E M F N D S - - - Q Y F G Y T E V G N
```

EP 0 511 306 B1

## FIGURE 1 (continued)

```
                    210             220             230             240             250
SEA    H T S T E P S V N Y D L  F G A Q G Q Y S N T - - L L R I Y R D N K T I N S E - N M H I D I Y L Y T S
SEE    H S S E G S T V S Y D L  F D A Q G Q Y P D T - - L L R I Y R D N K T I N S E - N L H I D L Y L Y T T
SED    D S S D G S K V S Y D L  F D V K G D F P E - - K Q L R I Y S D N K T L S T E - H L H I D I Y L Y E K
SEB    I E N E - N S F W Y D M  M P A P G D K F D Q S K Y L M M Y N D N K M V D S K - D V K I E V Y L T T K
SEC1   I E N N G N T F W Y D M  M P A P G D K F D Q S K Y L M M Y N D N K T V D S K - S V K I E V H L T T K
SEC3   I E N N G N T F W Y D M  M P A P G D K F D Q S K Y L M M Y N D N K T V D S K - S V K I E V H L T T K
SPE A  I P K N K E S F W F D F  F P E P - E F T - Q S K Y L M I Y K D N E T L D S N - T S Q I E V Y L T T K
SPE C  G T K D G K H E Q I D L  F D S P N E G T - R S D I F A K Y K D N R I I N M K N F S H F D I Y L - E K
TSST-1 T M N D G S T Y Q S D L  S K K F - E Y N - T E K P P I N I D E I K T I E A E I N *
ETA    S G S G I F N S N G E L  V G I H S S - - - - - - - - - - - - - - - - K V S H L D R E H Q I N Y G V G I
ETB    S G S G I F N L K G E L  I G I H S G K G G Q H N L P I G V F F N R K I S S L Y S V D N T - F G D T L
```

```
               260
SEA    *
SEE    *
SED    *
SEB    K K *
SEC1   N G *
SEC3   N G *
SPE A  *
SPE C  *
TSST-1
ETA    G N Y V - K R I I N E K N E *
ETB    G N D L K K R A K L D K *
```

EP 0 511 306 B1

FIGURE 2

```
           10        20              30       40        50
STR-PKPSQLQRSNLVKTFKIYIFFMRVTL-----VTHENVKSVDQLLSHDLIYNVS--
  :   ·:::   :   :    : :      : : :    :  :::::      ::::  :
ESQPDPKPDELHKSS--K-FTGLMENMKV-LYNNDHVSAINVKSINEFF--DLIYLYSIK
       10        20        30        40          50


           60        70        80        90
----GPNYDKLKTELKNQEMATLFKDKNVDIYGVEYYHLCYLC--------ENAERSAC
    : :::   : ::   :    ::: ::  :   ::   ::        ::   :  :
DTKLG-NYDNVRVEFKNKDLADKYKDKYVDVFGANYYQ-CYFSKKTNNIDSHENTKRKTC
      60        70        80        90        100       110


 100       110           120       130       140        150
LYGGVTNHEGNHLEIPKK----IVVKVSIDGIQSLSFDIEQIKNGNCSRIS-YTVRKYLT
 ::::::  :   : :     :      : : :  ::   ::::     :       :  : ::
MYGGVTEHGNNQLD---KYYRSITVRVFEDGKNLLSFDVQTNKKKVTAEQLDYLTRHYLV
     120       130       140       150       160


       160       170       180       190       200
DNKQLYTNGPSKYETGYIKFIPKNKESFWFDFFPEPE--FTQSKYLMIYKDNETLDSNTS
 ::  ::    :  :::::::::: :  ::: : :  :    : ::::::: :       ::
KNKKLYEFNNSPYETGYIKFIE-NENSFWYDMMPAPGNKFDQSKYLMMYNNDKMVDSKDV
170       180       190       200       210       220


       220
QIEVYLTTK
 :::::::::
KIEVYLTTKKK
 230
```

T CELL

FIG. 3